# EUROPEAN PATENT SPECIFICATION

(11) **EP 3 485 859 B1**
(45) Date of publication and mention of the grant of the patent: **24.02.2021**
(21) Application number: 16915114.9
(22) Date of filing: 31.08.2016
(51) Int. Cl.: A61F 13/15, A61F 13/49

(54) **METHOD AND DEVICE FOR MANUFACTURING SHEET-LIKE MEMBER FOR ABSORBENT ARTICLE**
VERFAHREN UND VORRICHTUNG ZUR HERSTELLUNG EINES FLÄCHENFÖRMIGEN ELEMENTS FÜR EINEN SAUGFÄHIGEN ARTIKEL
PROCÉDÉ ET DISPOSITIF DE FABRICATION D'ÉLÉMENT DE TYPE FEUILLE POUR UN ARTICLE ABSORBANT

(43) Date of publication of application: 22.05.2019
(73) Proprietor: Unicharm Corporation, Ehime 799-0111 (JP)
(72) Inventor: ISHIKAWA, Shinichi, Kanonji-shi Kagawa 769-1602 (JP); HANO, Shodai, Kanonji-shi Kagawa 769-1602 (JP); ISHIKAWA, Yoshihide, Kanonji-shi Kagawa 769-1602 (JP)
(74) Representative: Dolleymores
(86) International application number: PCT/JP2016/075489
(87) International publication number: WO 2018/042553

(56) References cited:
- EP-A1- 2 830 557
- WO-A1-96/10979
- JP-A- H03 251 245
- JP-A- H03 280 951
- JP-A- 2009 061 127
- JP-A- 2009 061 127
- JP-A- 2014 221 122
- JP-A- 2015 186 563
- JP-A- 2015 192 862
- JP-A- 2016 112 242
- JP-B1- 5 779 737

## Description

### [Technical Field]

The present invention relates to a method and a device for manufacturing a sheet-like member for an absorbent article such as a disposable diaper.

### [Background Art]

Conventionally, a sheet-like member is used when manufacturing a disposable diaper, which is known as an absorbent article for absorbing an excreted fluid such as urine. The sheet-like member includes a high-stretch region and a low-stretch region that are side-by-side in a predetermined direction, and the low-stretch region has lower stretchability in the predetermined direction than the high-stretch region.

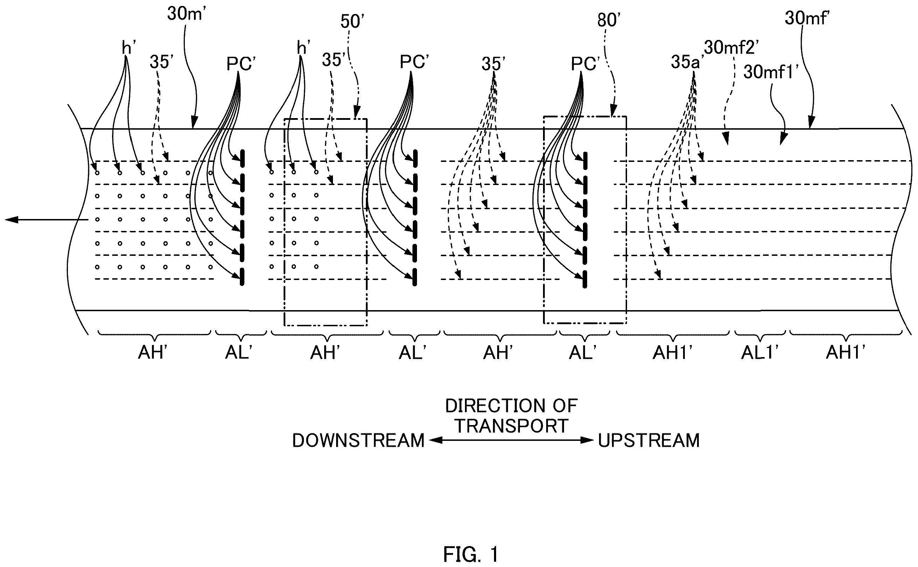

FIG. 1 is a schematic diagram showing an example of a process for producing a sheet-like member 30m'.

As shown in FIG. 1, first, a composite sheet 30mf' , which is the material for constituting the sheet-like member 30m' , is transported in a direction of transport. Here, this composite sheet 30mf' includes a first nonwoven fabric sheet 30mf1', a second nonwoven fabric sheet 30mf2', and elastic-string continuous bodies 35a', 35a'.... They are continuous in the direction of transport. The elastic-string continuous bodies 35a', 35a'... serve as elastic members, are inserted between the first nonwoven fabric sheet 30mf1' and the second nonwoven fabric sheet 30mf2' in a state of being stretched in the direction of transport, and are fixed in at least a region AH1' that corresponds to a high-stretch region AH'.

When this composite sheet 30mf' passes a position corresponding to a cutter device 80' in the manufacturing line, the device 80' cuts the elastic-string continuous bodies 35a', 35a'... by pressing cutter blades (not shown) at a predetermined position PC' in a region AL1' that corresponds to a low-stretch region AL' in the composite sheet 30mf', and as a result, the low-stretch region AL' and the high-stretch region AH' are formed side-by-side in the direction of transport in the sheet 30mf'. Specifically, due to this cutting, the cut elastic strings 35' and the portions of the elastic-string continuous bodies 35a' located upstream thereof contract toward the region AH1' corresponding to the high-stretch region AH'. Therefore, no elastic strings 35' are present in the region AL1' corresponding to the low-stretch region AL', whereas elastic strings 35' are present in the region AH1' corresponding to the high-stretch region AH'. As a result, the low-stretch region AL' and the high-stretch region AH' are formed in the composite sheet 30mf1'.

### [Citation List]

### [Patent Literature]

[PTL 1] Japanese Patent No. 5830624

WO 2013/148381 A1 relates to methods and apparatuses for assembling absorbent articles, each including a chassis connected with front and back elastic belts. An elastic laminate may be formed by continuously bonding elastic strands between a first continuous substrate layer and a second continuous substrate layer. The elastic strands are then intermittently severed in light-bond regions of the elastic laminate.

JP2009 061127 A discloses a diaper having a belt member including a plurality of air holes penetrating in the thickness direction.

### [Summary of Invention]

### [Technical Problem]

Also, as shown in FIG. 1, there are cases where multiple hole portions h' are formed in the high-stretch region AH' of the composite sheet 30mf' in order to form a high-stiffness portion and a low-stiffness portion or form air holes in the high-stretch region AH', for example. These hole portions h' are formed by pressing press-in members (not shown) such as pin members into the composite sheet 30mf' in the thickness direction thereof. It is conceivable for a hole-portion forming device 50', which is located downstream of the cutter device 80' in the direction of transport, to perform forming these hole portions h' after the cutter device 80' performs cutting the elastic-string continuous bodies 35a'.

However, when the elastic strings 35' that were cut by the cutter blades of the cutter device 80' and the portions of the elastic-string continuous bodies 35a' located upstream thereof contract toward the region AH1' corresponding to the high-stretch region AH', if the elastic strings 35' and the elastic-string continuous bodies 35a' are weakly fixed to the region AH1', there is a risk that the cut and contracting elastic strings 35' and elastic-string continuous bodies 35a' will intrude a large amount into the region AH1'. There is also a similar risk that the contracting elastic strings 35' and elastic-string continuous bodies 35a' will deviate. If this occurs, there is a risk that, due to the deviation, the elastic strings 35' and the elastic-string continuous bodies 35a' will move to positions where the hole portions h' are to be formed in the region AH1'.

In this case, the press-in members can possibly be pressed into the elastic strings 35' along with the nonwoven fabric sheets 30mf1' and 30mf2' of the composite sheet 30mf' when the hole portions h' are formed. As a result, the elastic strings 35' can be seen through the hole portions h', and this state leads to a poor appearance.

The present invention was achieved in light of conventional problems such as that described above, and an object of the present invention is to achieve a favorable appearance for the sheet-like member by preventing elastic members such as the elastic strings from being visible through hole portions in the sheet-like member.

### [Solution to Problem]

A main aspect of the present invention for achieving the above-described aspect is a method for manufacturing a sheet-like member for an absorbent article from a composite sheet,
the sheet-like member having a high-stretch region and a low-stretch region side-by-side in a predetermined direction,
the low-stretch region having a lower stretchability in the predetermined direction than the high-stretch region,
a plurality of hole portions being formed in at least the high-stretch region,
the method including:
a transporting step of transporting the composite sheet in a direction of transport that is the predetermined direction,
   the composite sheet being constituted by a first nonwoven fabric sheet, a second nonwoven fabric sheet, and a plurality of elastic members,
      the first nonwoven fabric sheet being continuous in the predetermined direction,
      the second nonwoven fabric sheet being continuous in the predetermined direction and arranged overlapped with the first nonwoven fabric sheet in a thickness direction of the composite sheet,
      the plurality of elastic members being stretched in the predetermined direction and extending along the predetermined direction,
      the plurality of elastic members being inserted between the first nonwoven fabric sheet and the second nonwoven fabric sheet in a side-by-side arrangement in a CD direction and being fixed in at least a region corresponding to the high-stretch region,
      the CD direction being a direction that intersects the predetermined direction;
   a hole-portion forming step of forming the hole portions in the composite sheet in the region corresponding to the high-stretch region, and engaging the first nonwoven fabric sheet and the second nonwoven fabric sheet with each other in at least edge portions of the hole portions,
      the hole portions extending in the thickness direction,
      the forming of the hole portions being performed by pressing a press-in member in the thickness direction at positions between a pair of elastic members that are adjacent in the CD direction and that are in at least the region corresponding to the high-stretch region; and
   an elastic-member cutting step of cutting the plurality of elastic members in a region corresponding to the low-stretch region, forming the high-stretch region and the low-stretch region side-by-side in the direction of transport in the composite sheet,
   the cutting being performed at a position that is downstream of a hole-portion formation position in the direction of transport, the elastic members being prevented from being visible through the hole portions, and the hole-portion formation position being a position at which formation of the hole portions in the hole-portion forming step is performed.

Further, a device for manufacturing a sheet-like member for an absorbent article from a composite sheet,
the sheet-like member having a high-stretch region and a low-stretch region side-by-side in a predetermined direction,
the low-stretch region having a lower stretchability in the predetermined direction than the high-stretch region,
a plurality of hole portions being formed in at least the high-stretch region,
the device including:
a transporting device for transporting the composite sheet in a direction of transport that is the predetermined direction,
   the composite sheet being constituted by a first nonwoven fabric sheet, a second nonwoven fabric sheet, and a plurality of elastic members,
   the first nonwoven fabric sheet being continuous in the predetermined direction,
   the second nonwoven fabric sheet being continuous in the predetermined direction and arranged overlapped with the first nonwoven fabric sheet in a thickness direction of the composite sheet,
   the plurality of elastic members being stretched in the predetermined direction and extending along the predetermined direction,
   the plurality of elastic members being inserted between the first nonwoven fabric sheet and the second nonwoven fabric sheet in a side-by-side arrangement in a CD direction and being fixed in at least a region corresponding to the high-stretch region,
   the CD direction being a direction that intersects the predetermined direction;
a hole-portion forming device for forming the hole portions in the composite sheet in the region corresponding to the high-stretch region, and engaging the first nonwoven fabric sheet and the second nonwoven fabric sheet with each other in at least edge portions of the hole portions,
   the hole portions extending in the thickness direction,
   the forming of the hole portions being performed by pressing a press-in member in the thickness direction at positions between a pair of elastic members that are adjacent in the CD direction and that are in at least the region corresponding to the high-stretch region; and
an elastic-member cutting device for cutting the plurality of elastic members in a region corresponding to the low-stretch region, forming the high-stretch region and the low-stretch region side-by-side in the direction of transport in the composite sheet,
   the cutting being performed at a position that is downstream of a hole-portion formation position in the direction of transport,
   the elastic members being prevented from being visible through the hole portions, and the hole-portion formation position being a position at which the hole portions are formed by the hole-portion forming device.

Features of the present invention other than the above will become clear by reading the description of the present specification with reference to the accompanying drawings.

### [Advantageous Effects of Invention]

According to the present invention, it is possible to achieve a favorable appearance for a sheet-like member by preventing elastic members such as elastic strings from being visible through hole portions in the sheet-like member.

### [Brief Description of the Drawings]

FIG. 1 is a schematic diagram showing an example of a process for producing a sheet-like member 30m' of a reference example.
FIG. 2 is a schematic perspective view of a pants-shaped state of a three-piece type of disposable diaper 1, which is one example of an absorbent article, as viewed from a front side.
FIG. 3 is a schematic plan view of the diaper 1 in an unfolded state, as viewed from a skin side.
FIG. 4 is a cross-sectional view taken along IV-IV in FIG. 3.
FIG. 5 is a schematic plan view of a front band member 31, as viewed from a non-skin side in a thickness direction.
In FIG. 6, FIG. 6A is a schematic enlarged view of air holes h according to a first embodiment as viewed from a non-skin side in a thickness direction, FIG. 6B is a cross-sectional view taken along B-B in FIG. 6A, and FIG. 6C is a schematic enlarged view of air holes h in a comparative example as viewed from the non-skin side.
FIG. 7 is a schematic plan view of a manufacturing line for illustrating various types of processing performed in the manufacturing line.
FIG. 8A is a schematic plan view of an intermediate product 1m immediately before being transported to an air-hole forming step S50 position.
FIG. 8B is a schematic plan view of an intermediate product 1m after passing through the air-hole forming step S50.
In FIG. 9, FIG. 9A is a schematic plan view of front-band-member continuous bodies 31a immediately before being transported to a front-elastic-string cutting step S80 position, and FIG. 9B is a schematic plan view of the front-band-member continuous bodies 31a during and after passing through the step S80.
FIG. 10 is a schematic side view of an air-hole forming device 50, in which portions of the configurations are shown in a longitudinal cross-sectional view.
FIG. 11 is a schematic enlarged view taken along arrows XI-XI in FIG. 10.
FIG. 12 is a diagram illustrating an arrangement pattern of pin members 55p on the outer circumferential surface of an intermediate roller 55.
FIG. 13 is a diagram illustrating an arrangement pattern of hole portions 51h in the outer circumferential surface of an upstream roller 51.
In FIG. 14, FIG. 14A is an enlarged view of a portion XIVa in FIG. 12, and FIG. 14B is a view taken along arrows B-B in FIG. 14A.
In FIG. 15, FIG. 15A is a schematic side view of a front-side cutter device 80, and FIG. 15B is a view taken along arrows B-B in FIG. 15A.
In FIG. 16, FIGS. 16A and 16B are illustrative diagrams of preferable relative positional relationships between marks TC and air holes h.
FIG. 17 is a schematic illustrative diagram of a manufacturing method of a second embodiment.
FIG. 18 is a schematic illustrative diagram of a manufacturing method of a third embodiment.

### [Description of Embodiments]

At least the following matters will become clear with the description of this specification and the attached drawings.

A method for manufacturing a sheet-like member for an absorbent article from a composite sheet,
the sheet-like member having a high-stretch region and a low-stretch region side-by-side in a predetermined direction,
the low-stretch region having a lower stretchability in the predetermined direction than the high-stretch region,
a plurality of hole portions being formed in at least the high-stretch region,
the method including: a transporting step of transporting the composite sheet in a direction of transport that is the predetermined direction,
   the composite sheet being constituted by a first nonwoven fabric sheet, a second nonwoven fabric sheet, and a plurality of elastic members,
   the first nonwoven fabric sheet being continuous in the predetermined direction,
   the second nonwoven fabric sheet being continuous in the predetermined direction and arranged overlapped with the first nonwoven fabric sheet in a thickness direction of the composite sheet,
   the plurality of elastic members being stretched in the predetermined direction and extending along the predetermined direction,
   the plurality of elastic members being inserted between the first nonwoven fabric sheet and the second nonwoven fabric sheet in a side-by-side arrangement in a CD direction and being fixed in at least a region corresponding to the high-stretch region,
   the CD direction being a direction that intersects the predetermined direction;
a hole-portion forming step of forming the hole portions in the composite sheet in the region corresponding to the high-stretch region, and engaging the first nonwoven fabric sheet and the second nonwoven fabric sheet with each other in at least edge portions of the hole portions,
   the hole portions extending in the thickness direction,
   the forming of the hole portions being performed by pressing a press-in member in the thickness direction at positions between a pair of elastic members that are adjacent in the CD direction and that are in at least the region corresponding to the high-stretch region; and
an elastic-member cutting step of cutting the plurality of elastic members in a region corresponding to the low-stretch region, forming the high-stretch region and the low-stretch region side-by-side in the direction of transport in the composite sheet,
   the cutting being performed at a position that is downstream of a hole-portion formation position in the direction of transport, the elastic members being prevented from being visible through the hole portions, and
   the hole-portion formation position being a position at which formation of the hole portions in the hole-portion forming step is performed.

According to this method for manufacturing a sheet-like member for an absorbent article, when the elastic members are cut, the cut elastic members contract toward the region that corresponds to the high-stretch region, and it is possible to prevent the elastic members from being visible through the hole portions after contraction. In other words, if the strength of the fixing of the elastic members to the region is low, there is a risk that the cut and contracting elastic members will intrude a large amount into the region. But the hole portions are formed in the region, and the first nonwoven fabric sheet and the second nonwoven fabric sheet are engaged with each other in the edge portions of the hole portions. Accordingly, even if the cut and contracting elastic members deviate in the CD direction and attempt to approach the hole portions, this engagement of the first nonwoven fabric sheet and the second nonwoven fabric sheet suppresses further movement of the elastic members toward the hole portions . Accordingly, it is possible to prevent the elastic members from being visible through the hole portions after the elastic members contract, and as a result, it is possible to achieve a favorable appearance for the sheet-like member.

In such a method for manufacturing a sheet-like member for an absorbent article, it is desirable
that the composite sheet has a base sheet that is continuous in the direction of transport,
that the base sheet has a first portion that is to become the first nonwoven fabric sheet and a second portion that is to become the second nonwoven fabric sheet, the first portion and the second portion being integrated as a single piece and adjacent in the CD direction, and
that the method further comprises a folding step of, at a position that is upstream in the direction of transport of the hole-portion formation position of the hole-portion forming step, folding the base sheet in the CD direction at a folding position that is a boundary position between the first portion and the second portion such that the first nonwoven fabric sheet and the second nonwoven fabric sheet become overlapped.

According to this method for manufacturing a sheet-like member for an absorbent article, the first portion and the second portion can be firmly engaged with each other in the edge portions of the hole portions. This will be described in detail below. First, when the first portion and the second portion are overlaid on each other, if characteristics such as the density (g/cm³) of the nonwoven fabric sheets are the same or similar at the surfaces that come into contact with each other, the constituent fibers more easily become entangled in the edge portions of the hole portions when the hole portions are formed. As a result, the first portion and the second portion become engaged with each other with a high degree of engagement in the edge portions. There are also cases where characteristics are different between the two surfaces of the base sheet, but in such cases, according to the above configuration, when the first portion and the second portion are folded at the folding positions, portions of the same one of the two surfaces of the base sheet face each other and come into contact with each other. Accordingly, surfaces of the first portion and the second portion that have the same characteristics as each other come into contact with each other, thus making it possible for the first portion and the second portion to become firmly engaged with each other at these surfaces in the edge portions of the hole portions.

In such a method for manufacturing a sheet-like member for an absorbent article, it is desirable
that letting a third portion be a portion in which the first portion and the second portion are integrated as a single piece,
   the base sheet has the third portion on each of two sides in the CD direction, and
that, in the folding step, the base sheet is folded at the folding position that is located on each of the two sides in the CD direction.

According to this method for manufacturing a sheet-like member for an absorbent article, two members having a two-layer structure and including the first nonwoven fabric sheet and the second nonwoven fabric sheet that are overlaid on each other, can be formed from one base sheet. Accordingly, there is no need to provide two of the same device in order to form these two members with a two-layer structure. For example, there is no need to provide two feeding devices for feeding nonwoven fabric from original fabric, such as one for the nonwoven fabric for the first layer and another one for the nonwoven fabric for the second layer, that is to say, it is sufficient to provide one feeding device for the base sheet. As a result, it is possible to reduce the number of devices and reduce the equipment cost.

In such a method for manufacturing a sheet-like member for an absorbent article, it is desirable
that the region corresponding to the high-stretch region has a fixing portion for fixing the elastic members,
that the first nonwoven fabric sheet and the second nonwoven fabric sheet are fixed to each other in the fixing portion, and
that besides the fixing portion and the edge portions of the hole portions, the region corresponding to the high-stretch region does not have a fixing portion that fixes the first nonwoven fabric sheet and the second nonwoven fabric sheet to each other.

According to this method for manufacturing a sheet-like member for an absorbent article, the first nonwoven fabric sheet and the second nonwoven fabric sheet are fixed not only by the fixing portion, but also by the edge portions. They can therefore be fixed firmly. Also, in the high-stretch region, it is possible to eliminate fixing portions that are solely for fixing the first nonwoven fabric sheet and the second nonwoven fabric sheet, thus making it possible to manufacture a softer sheet-like member.

In such a method for manufacturing a sheet-like member for an absorbent article, it is desirable
that the fixing portion is an adhesive applied to the elastic members, and
that the adhesive is not provided in the edge portions.

According to this method for manufacturing a sheet-like member for an absorbent article, an adhesive is not provided in the edge portions of the hole portions, and the first nonwoven fabric sheet and the second nonwoven fabric sheet are fixed to each other by only engagement between the first and second nonwoven fabric sheets in the edge portions. Accordingly, the manufactured sheet-like member can be made even softer.

In such a method for manufacturing a sheet-like member for an absorbent article, it is desirable that
the method further comprises an absorbent-main-body fixing step of fixing the absorbent main body for the absorbent article to the low-stretch region of the composite sheet, at a position that is downstream in the direction of transport of a cutting position,
the cutting position being a position at which the elastic members are cut in the elastic-member cutting step.

According to this method for manufacturing a sheet-like member for an absorbent article, the absorbent main body is fixed to the low-stretch region of the composite sheet after the hole-portion forming step and the elastic-member cutting step. Accordingly, it is possible to reliably prevent a problem that can occur if this order is reversed, that is to say a problem that when the hole portions are formed in the composite sheet and the elastic members are cut after the absorbent main body is fixed, the formation of the hole portions and the cutting are performed on the absorbent main body as well, thus damaging the absorbent main body.

In such a method for manufacturing a sheet-like member for an absorbent article, it is desirable
that the method further comprises a continuous-sheet fixing step of fixing a continuous sheet that is continuous in the predetermined direction to the composite sheet, at a position that is downstream in the direction of transport of the hole-portion formation position of the hole-portion forming step, and
that, in the continuous-sheet fixing step,
the continuous sheet is overlaid in the thickness direction so as to cover several of the hole portions.

According to this method for manufacturing a sheet-like member for an absorbent article, the continuous sheet is overlaid on the composite sheet so as to cover several hole portions. Accordingly, even if the elastic members are located in the hole portions, the elastic members can be hidden by the continuous sheet so as to not be visible. Accordingly, the sheet-like member can be given a favorable appearance.

In such a method for manufacturing a sheet-like member for an absorbent article, it is desirable
that the elastic members are cut by cutter blades being pressed into the composite sheet, and a cutting mark is formed in the composite sheet, and
that the cutting is performed such that the mark is not overlapped in the CD direction with, among the plurality of hole portions, a pair of hole portions that are located closest to the mark on two sides in the direction of transport.

According to this method for manufacturing a sheet-like member for an absorbent article, it is possible to prevent a problem that the shape of the hole portions is deformed by the marks when the elastic members are cut by the cutter blades.

In such a method for manufacturing a sheet-like member for an absorbent article, it is desirable that
a size of the mark in the direction of transport is smaller than a distance in the direction of transport between, among the plurality of hole portions, the pair of hole portions that are located closest to the mark on the two sides in the direction of transport.

According to this method for manufacturing a sheet-like member for an absorbent article, it is possible to more reliably prevent a problem that the shape of the hole portions is deformed by the marks when the elastic members are cut by the cutter blades.

In such a method for manufacturing a sheet-like member for an absorbent article, it is desirable that
the hole portions are through holes that pass through the composite sheet in the thickness direction.

According to this method for manufacturing a sheet-like member for an absorbent article, the hole portions are through holes, thus improving breathability in the high-stretch region of the sheet-like member.

In such a method for manufacturing a sheet-like member for an absorbent article, it is desirable that
the hole portions are bottomed holes that each have a bottom portion.

According to this method for manufacturing a sheet-like member for an absorbent article, the hole portions are bottomed holes, thus making it possible to reliably prevent the elastic members from being visible through the hole portions.

A device for manufacturing a sheet-like member for an absorbent article from a composite sheet,
the sheet-like member having a high-stretch region and a low-stretch region side-by-side in a predetermined direction,
the low-stretch region having a lower stretchability in the predetermined direction than the high-stretch region,
a plurality of hole portions being formed in at least the high-stretch region,
the device including:
   a transporting device for transporting the composite sheet in a direction of transport that is the predetermined direction,
      the composite sheet being constituted by a first nonwoven fabric sheet, a second nonwoven fabric sheet, and a plurality of elastic members,
      the first nonwoven fabric sheet being continuous in the predetermined direction,
      the second nonwoven fabric sheet being continuous in the predetermined direction and arranged overlapped with the first nonwoven fabric sheet in a thickness direction of the composite sheet,
      the plurality of elastic members being stretched in the predetermined direction and extending along the predetermined direction,
      the plurality of elastic members being inserted between the first nonwoven fabric sheet and the second nonwoven fabric sheet in a side-by-side arrangement in a CD direction and being fixed in at least a region corresponding to the high-stretch region,
      the CD direction being a direction that intersects the predetermined direction;
   a hole-portion forming device for forming the hole portions in the composite sheet in the region corresponding to the high-stretch region, and engaging the first nonwoven fabric sheet and the second nonwoven fabric sheet with each other in at least edge portions of the hole portions,
      the hole portions extending in the thickness direction,
      the forming of the hole portions being performed by pressing a press-in member in the thickness direction at positions between a pair of elastic members that are adjacent in the CD direction and that are in at least the region corresponding to the high-stretch region; and
   an elastic-member cutting device for cutting the plurality of elastic members in a region corresponding to the low-stretch region, forming the high-stretch region and the low-stretch region side-by-side in the direction of transport in the composite sheet,
   the cutting being performed at a position that is downstream of a hole-portion formation position in the direction of transport, the elastic members being prevented from being visible through the hole portions, and the hole-portion formation position being a position at which the hole portions are formed by the hole-portion forming device.

According to this device for manufacturing a sheet-like member related to an absorbent article, it is possible to achieve actions and effects similar to those of the manufacturing method described above.

### First Embodiment

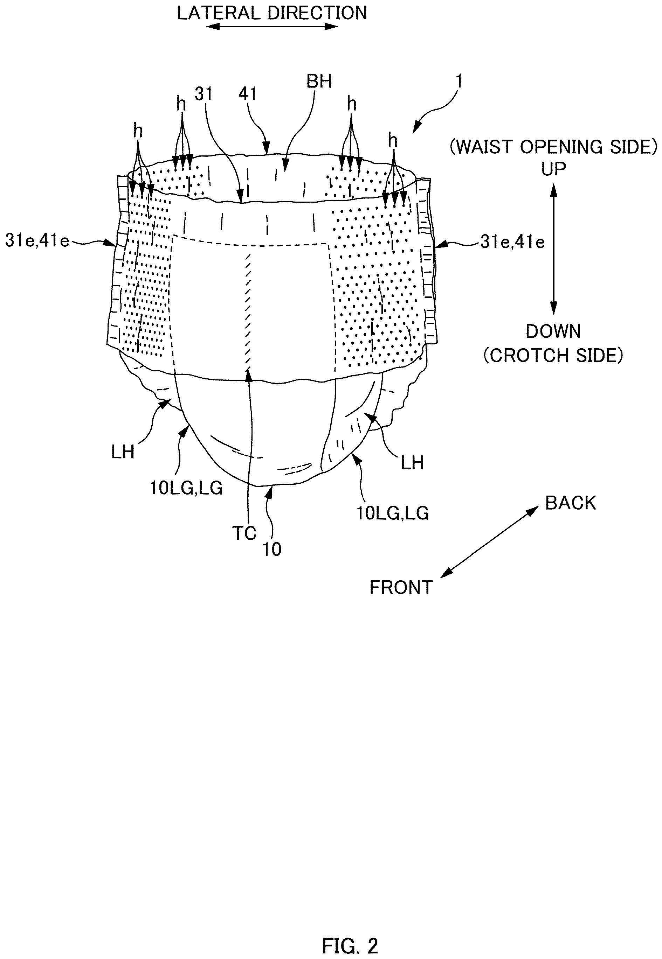

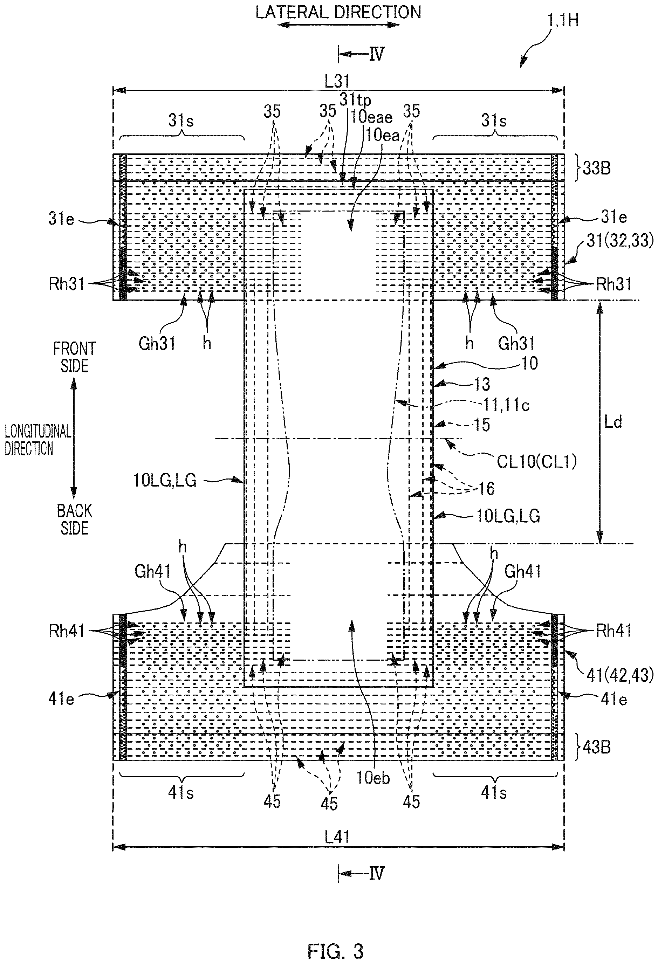

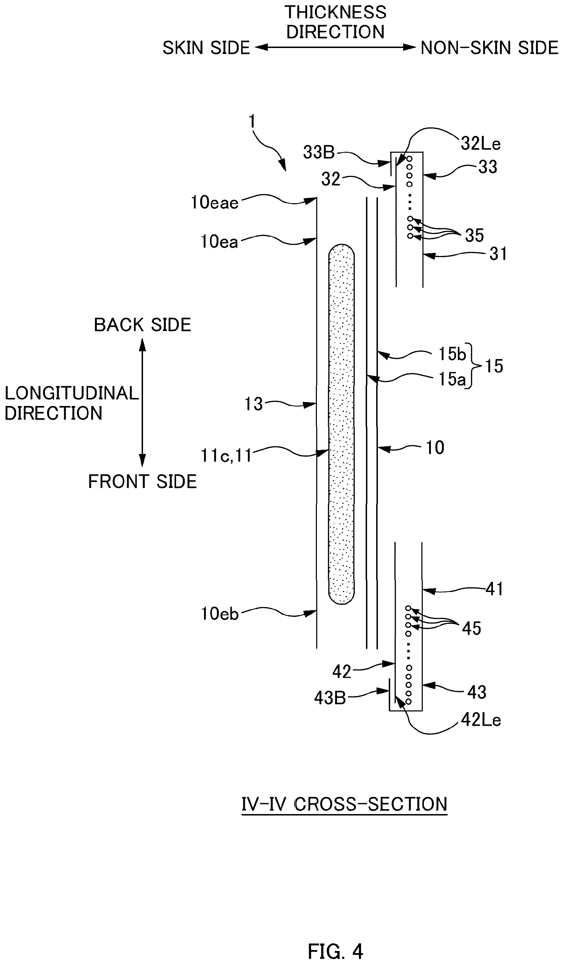

A method and device according to the first embodiment of the present invention for manufacturing a sheet-like member for an absorbent article are used in a manufacturing line for manufacturing a disposable diaper 1, which is one example of the absorbent article. FIG. 2 is a schematic perspective view of a pants-shaped state of a three-piece type of diaper 1, which is one example of the disposable diaper 1, as viewed from a front side. Also, FIG. 3 is a schematic plan view of the diaper 1 in an unfolded state, as viewed from the skin side, and FIG. 4 is a cross-sectional view taken along IV-IV in FIG. 3.

In the pants-shaped state shown in FIG. 2, the diaper 1 has an up-down direction, a lateral direction, and a front-back direction as three directions that are orthogonal to each other. In the following, the upper side and the lower side in the up-down direction in the pants-shaped state will also be respectively called the "waist opening side" and the "crotch side", and the front side and the back side in the front-back direction will also be respectively called the "front side" and the "back side". Also, in the unfolded state shown in FIGS. 3 and 4, the diaper 1 has a longitudinal direction, a lateral direction, and a thickness direction as three directions that are orthogonal to each other. In the following, one side and the other side in the longitudinal direction in the unfolded state will also be respectively called the "front side" and the "back side", and one side and the other side in the thickness direction will also be respectively called the "skin side" and the "non-skin side". Note that the lateral direction has the same meaning in the pants-shaped state and the unfolded state. Also, the longitudinal direction in the unfolded state conforms to the up-down direction in the pants-shaped state, and the thickness direction in the unfolded state conforms to the front-back direction in the pants-shaped state. Furthermore, in the unfolded state shown in FIGS. 3 and 4, the diaper 1 is shown in a state of being stretched out to the extent that no contractive force whatsoever is exerted by elastic strings 16, 35, and 45 that serve as later-described elastic members for giving stretchability to the diaper 1.

Because the diaper 1 is a so-called three piece type of diaper, in the unfolded state shown in FIG. 3, it has an absorbent main body 10 for absorbing excrement as a first component, a front band member 31 as a second component, and a back band member 41 as a third component. Specifically, in the state where the front band member 31 and the back band member 41 are arranged parallel to each other with a gap therebetween in the longitudinal direction, the absorbent main body 10 spans between the members 31 and 41, and end portions 10ea and 10eb in the longitudinal direction of the absorbent main body 10 are respectively joined and fixed to the closest ones out of the band members 31 and 41, thus having an approximately H-shaped appearance in a plan view.

In this approximately H-shaped unfolded state, the absorbent main body 10 is folded in two at a folding position that is a predetermined position CL10 (position corresponding to a central position CL1 of the diaper 1 in the longitudinal direction) in the longitudinal direction of the absorbent main body 10, and when the band members 31 and 41, which face each other in this folded state, are joined by welding in lateral end portions 31e and 41e, the band members 31 and 41 join together to form a ring shape, thus obtaining the diaper 1 in the pants-shaped state in which a waist opening BH and a pair of leg openings LH are formed as shown in FIG. 2.

Note that in the unfolded state shown in FIGS. 3 and 4, the absorbent main body 10 is approximately rectangular in a plan view. The lengthwise direction of the absorbent main body 10 conforms to the longitudinal direction. Also, the absorbent main body 10 includes an absorbent body 11, a top sheet 13 that covers the absorbent body 11 from the skin side, and a back sheet 15 that covers the absorbent body 11 from the non-skin side.

The absorbent body 11 has a liquid-absorbent absorbent core 11c, and a core-wrapping sheet that is made of tissue paper or the like (not shown) and covers the outer circumferential surface of the core 11c. The absorbent core 11c is a molded body that is made of a predetermined liquid absorbent material and is approximately hourglass-shaped in a plan view, as one example of a predetermined shape. Examples of the liquid absorbent material include liquid-absorbent fibers such as pulp fibers, and liquid-absorbent particles made of a superabsorbent polymer (so-called SAP) or the like.

The top sheet 13 is a liquid-permeable sheet that is made of nonwoven fabric and has a planar size according to which it projects from the absorbent body 11 in the longitudinal direction and the lateral direction. The back sheet 15 is also a sheet having a planar size according to which it projects from the absorbent body 11 in the longitudinal direction and the lateral direction, and one example is the double-layer structure laminate sheet 15 shown in FIG. 4. Specifically, this laminate sheet 15 has a liquid-impermeable leak-proof sheet 15a made of a polyethylene film (PE) or a polypropylene film (PP) on the skin side, and has an exterior sheet 15b made of nonwoven fabric on the non-skin side.

When the absorbent body 11 is sandwiched between the top sheet 13 and the back sheet 15, the portions of the sheets 13 and 15 that project from the absorbent body 11 in the longitudinal direction and the lateral direction are joined together by adhesion, welding, or the like to obtain a frame-like shape, thus forming the absorbent main body 10. Note that the exterior sheet 15b may be omitted, and the back sheet 15 may have only the leak-proof sheet 15a.

Also, as shown in this example in FIG. 3, portions 10LG of the absorbent main body 10 that are laterally outward of the absorbent body 11 may be provided with leg gathers LG that stretch and contract in the longitudinal direction. These leg gathers LG constitute portions of the leg openings LH. Also, the leg gathers LG are given stretchability by fixing elastic strings 16, which are elastic members that extend in the longitudinal direction, to the portions 10LG in a state of being stretched in the longitudinal direction. Note that in addition to these leg gathers LG, in order to prevent lateral leakage, so-called barrier cuffs (not shown) may be provided as leak-proof wall portions on the two lateral sides of the absorbent main body 10.

Also, as shown in FIG. 3, the front band member 31 is a sheet member that has an approximately rectangular shape in a plan view, and is made up of nonwoven fabric sheets 32 and 33. In this example, as shown in FIG. 4, the front band member 31 is formed by joining together the two overlapped nonwoven fabric sheets 32 and 33 with use of hot-melt adhesive. Also, as shown in FIGS. 3 and 4, the laterally central portion of the front band member 31 is overlaid on, from the non-skin side, and joined to a front longitudinal end portion 10ea of the absorbent main body 10.

Similarly to the front band member 31, the back band member 41 is also a sheet member that has an approximately rectangular shape in a plan view and is made up of nonwoven fabric sheets 42 and 43, and in this example, as shown in FIG. 4, the back band member 41 is formed by joining together the two overlapped nonwoven fabric sheets 42 and 43 with use of a hot-melt adhesive. Also, as shown in FIGS. 3 and 4, the laterally central portion of the back band member 41 is overlaid on, from the non-skin side, and joined to a back longitudinal end portion 10eb of the absorbent main body 10.

Note that the content described below is the same for both the front band member 31 and the back band member 41. For this reason, the following describes only the front band member 31 as a representative for both, and corresponding portions of the back band member 41 are simply indicated in parentheses.

As shown in FIGS. 3 and 4, multiple elastic strings 35, 35... (45, 45...), which are elastic members that extend in the lateral direction, are inserted side-by-side in the longitudinal direction between the two nonwoven fabric sheets 32 and 33 (42 and 43) of the front band member 31 (41), and are joined and fixed to the nonwoven fabric sheets 32 and 33 (42 and 43) with use of a hot-melt adhesive, in a state of being stretched in the lateral direction. Accordingly, the front band member 31 (41) is given stretchability in the lateral direction.

Also, in this example, as shown in FIG. 3, the elastic strings 35 (45) are non-continuous in at least a portion (e.g., the laterally central portion) of the front band member 31 (41) that is overlapped with the absorbent core 11c, in order to prevent the formation of wrinkles in the absorbent body 11 for example. Accordingly, stretchability is not given to this portion.

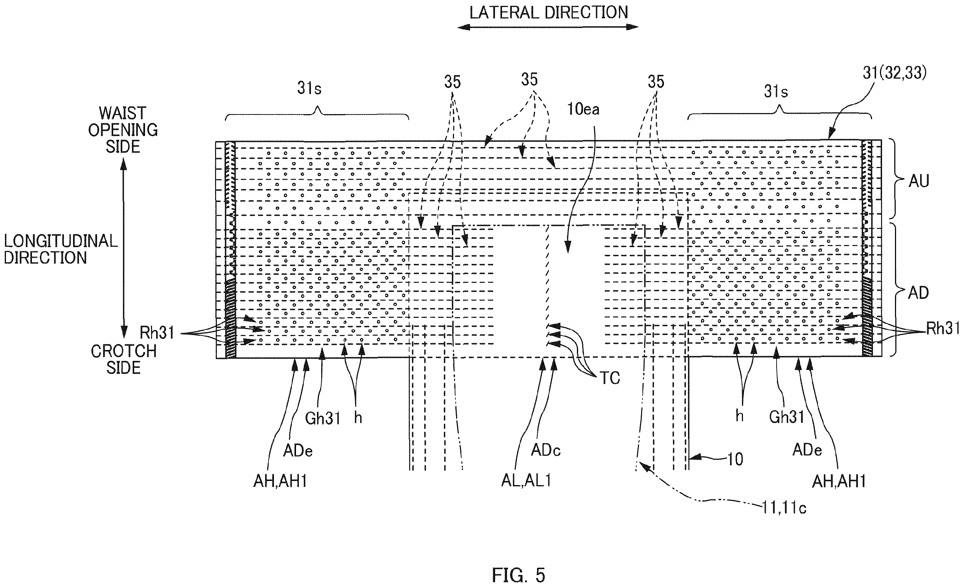

Specifically, as shown in the schematic plan view of the front band member 31 as viewed from the non-skin side in the thickness direction in FIG. 5, if the front band member 31 (41) is divided into two regions AU and AD in the longitudinal direction, with "upper region AU" referring to the region AU located on the waist opening BH side in the longitudinal direction, and "lower region AD" referring to the region AD located on the crotch side, then the absorbent core 11c of the absorbent main body 10 is substantially not overlapped with the upper region AU. For this reason, the upper region AU is given stretchability over substantially the entire length in the lateral direction. In other words, the elastic strings 35, 35... (45, 45...) are provided extending over substantially the entire length in the lateral direction in the upper region AU.

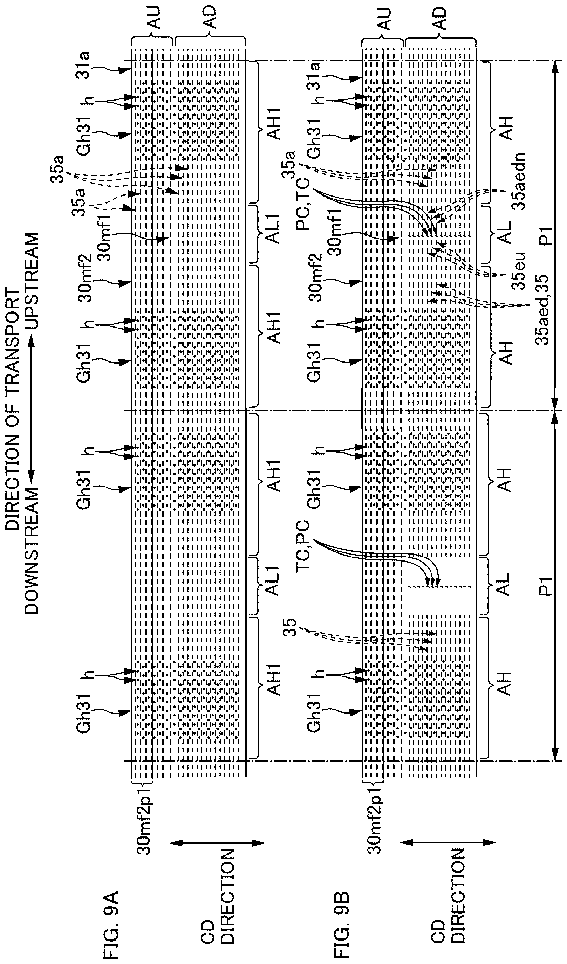

Meanwhile, in the lower region AD, the absorbent core 11c of the absorbent main body 10 is overlapped with a laterally central region ADc, and therefore the elastic strings 35, 35... are not arranged in the central portion of this central region ADc, thus forming a non-stretchy region AL (corresponding to a low-stretch region) having substantially no lateral stretchability in this central portion of the central region ADc. It should be noted that the absorbent core 11c is substantially not overlapped with two end regions ADe located on respective sides of the central region ADc, and therefore the elastic strings 35, 35... (45, 45...) are arranged in these end regions Ade, thus forming two stretchy regions AH (corresponding to a high-stretch region) that have higher stretchability than the non-stretchy region AL, in the two end regions ADe. Note that the non-stretchy region AL and the stretchy regions AH are formed in the lower region AD by cutting continuous bodies 35a (45a) of the elastic strings (see FIGS. 9A and 9B), which are arranged traversing the central portion in the lateral direction, the cutting being made in the central portion. This cutting will be described later.

Also, the fiber density of these elastic strings 35 (45) is 400 dtex to 1000 dtex for example, and one specific example of the elastic strings 35 (45) is LYCRA (registered trademark) . Note that there is no limitation whatsoever to this.

Furthermore, in this example, as shown in FIG. 4, the planar size of the nonwoven fabric sheet 33 (43), which is the one of the two nonwoven fabric sheets 32 and 33 (42 and 43) that is located on the non-skin side in the thickness direction, is a size according to which it projects longitudinally outward from the nonwoven fabric sheet 32 (42) that is located on the skin side. Also, the projecting portion of the nonwoven fabric sheet 33 (43) is folded back inward in the longitudinal direction, and this folded portion 33B (43B) covers a longitudinal end portion 32Le (42Le) of the nonwoven fabric sheet 32 (42) from the skin side, but there is no limitation whatsoever to this.

Also, as shown in FIGS. 3 and 5, in each of the two lateral sides of the front band member 31 (41), there is a portion 31s (41s) (hereinafter, also called a no-absorbent-body portion 31s (41s)) that includes the end region ADe and is not overlapped by the absorbent main body 10. In order to improve breathability in this no-absorbent-body portion 31s (41s), multiple air holes h (h) that pass through the no-absorbent-body portion 31s (41s) are formed discretely in a predetermined arrangement pattern, and these air holes h (h) form an air hole group Gh31 (Gh41), which is a group of air holes h. Specifically, in this example, in each of the no-absorbent-body portions 31s (41s), multiple air holes h, h... (h, h...) are formed side-by-side with a predetermined formation pitch at positions between pairs of elastic strings 35 (45) that are adjacent in the longitudinal direction, and therefore these air holes h, h... (h, h...) form air hole rows Rh31 (Rh41) that extend in the lateral direction. Also, pairs of air hole rows Rh31 (Rh41) that are adjacent in the longitudinal direction are shifted from each other in the lateral direction by half the formation pitch. Accordingly, the air holes h, h... (h, h...) form the air hole group Gh31 (Gh41) that has a substantially staggered arrangement. Note that there is no limitation whatsoever to this. For example, the air holes h, h... (h, h...) may be formed in a lattice arrangement.

Furthermore, in this example, the air holes h, h... are each formed with a target opening shape, which is a perfect circle having a diameter of 0.2 mm to 3 mm, but there are also air holes h, h... that are not a perfect circle due to formation precision problems. The diameter of such non-perfect circle air holes h varies according to the position on the circumference of the air hole h, but even these varying diameters fall in the range of 0.2 mm to 3 mm, for example. Accordingly, the air holes h can quickly exhibit anticipated breathability. It should be noted that the target opening shape of the air holes h is not limited in any way to the aforementioned perfect circle. For example, the target opening shape may be any polygon, such as an equilateral triangle or a square.

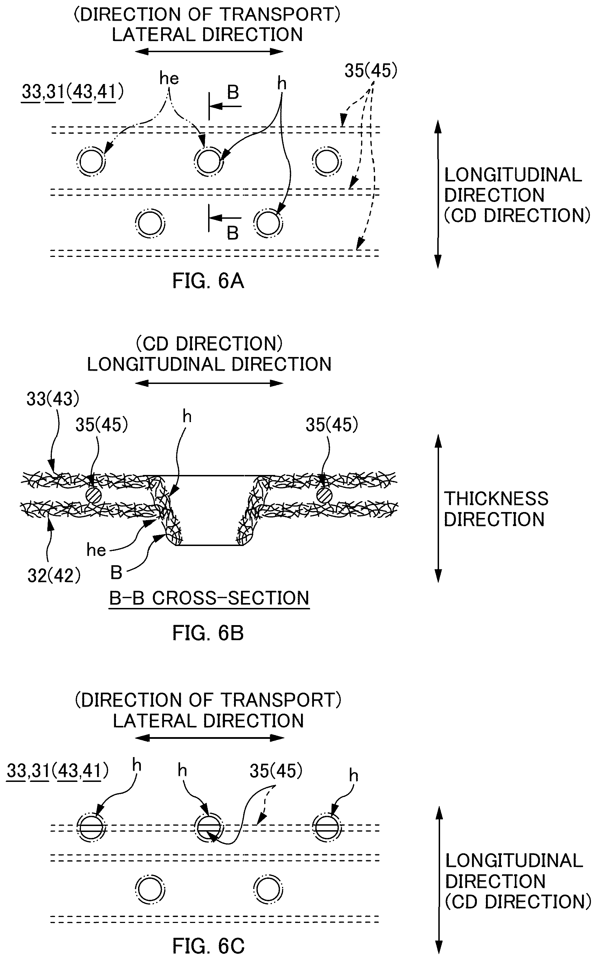

Also, FIG. 6A shows a schematic enlarged view of air holes has viewed from the non-skin side in the thickness direction, FIG. 6B shows a cross-sectional view taken along B-B in FIG. 6A, and as can be understood from FIGS. 6A and 6B, burrs B project from edge portions he of the air holes h, and the nonwoven fabric sheets 32 and 33 (42 and 43) are engaged with each other at the burrs B and in the vicinity thereof. Specifically, in these edge portions he, the nonwoven fabric sheets 32 and 33 (42 and 43) are engaged with each other due to the constituent fibers thereof becoming entangled with each other, for example. Intrusion of the elastic strings 35 (45) into the air holes h as shown in a comparative example in FIG. 6C is thus prevented by this engagement in the edge portions he, and the appearance is improved. Note that this will be described later.

Also, in this example, the two nonwoven fabric sheets 32 and 33 for the front band member 31 and the two nonwoven fabric sheets 42 and 43 for the back band member 41 are each made of spunbond nonwoven fabric. It should be noted that there is no limitation whatsoever to this, and it is possible to use a different type of nonwoven fabric such as SMS (spunbond/meltblown/spunbond) nonwoven fabric. Also, standalone fibers made of polypropylene (PP), which is a representative example of a thermoplastic resin, are used as the fibers that constitute the nonwoven fabric, but there is no limitation whatsoever to this. For example, standalone fibers made of another type of thermoplastic resin, such as polyethylene (PE), may be used, and furthermore, it is possible to use composite fibers that have a core-sheath structure including PE and PP, for example.

This diaper 1 is manufactured in a manufacturing line. In this manufacturing line, an intermediate product 1m of the diaper 1 is transported along a predetermined direction of transport (corresponding to a transporting step). During this transporting, various types of processing are performed on the intermediate product 1m, whereby each time processing is performed, the shape of the intermediate product 1m successively changes, and ultimately the diaper 1 shown in FIG. 2 is completed.

Note that in the following, the width direction of the manufacturing line is also called the "CD direction". Moreover, in this example, the CD direction conforms to the horizontal direction. In this manufacturing line, the direction of transport along which the intermediate product 1m is transported is any direction in a plane that is orthogonal to the CD direction. In other words, the direction of transport is a direction defined by both the vertical up-down direction and the horizontal front-back direction. Note that the terms "up-down direction" and "front-back direction" used here are directions that are different from and not directly related to the terms "up-down direction" and the "front-back direction" used in the above description of the diaper 1.

Also, the intermediate product 1m is transported by appropriate transporting devices such as a belt transporter and transporting rollers. Accordingly, unless particularly stated otherwise, the intermediate product 1m is assumed to be transported in the direction of transport by these transporting devices. Note that examples of belt transporters include a normal belt transporter that has a circulating endless belt as a transporting surface, and a suction belt transporter that includes an endless belt whose outer circumferential surface has a suction function.

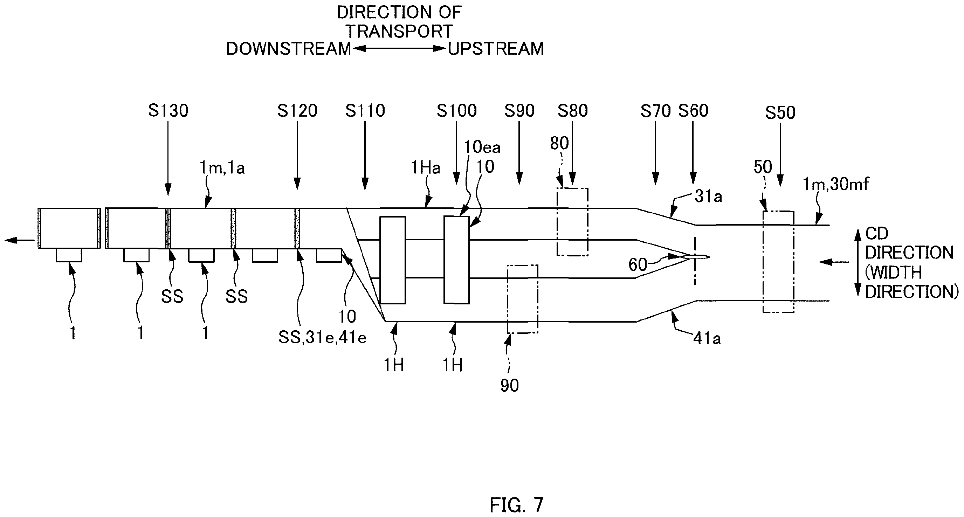

FIG. 7 is a schematic plan view of the manufacturing line for illustrating various types of processing performed in the manufacturing line. The manufacturing line has an air-hole forming step S50 (corresponding to a hole-portion forming step), a slitting step S60, a gap forming step S70, a front-elastic-string cutting step S80 (corresponding to an elastic-member cutting step), a back-elastic-string cutting step S90 (corresponding to an elastic-member cutting step), an absorbent-main-body attaching step S100 (corresponding to an absorbent-main-body fixing step), a folding step S110, a side sealing step S120, and a cutting step S130. The positions at which these steps S50 to S130 are performed are arranged side-by-side in the stated order from upstream to downstream in the direction of transport.

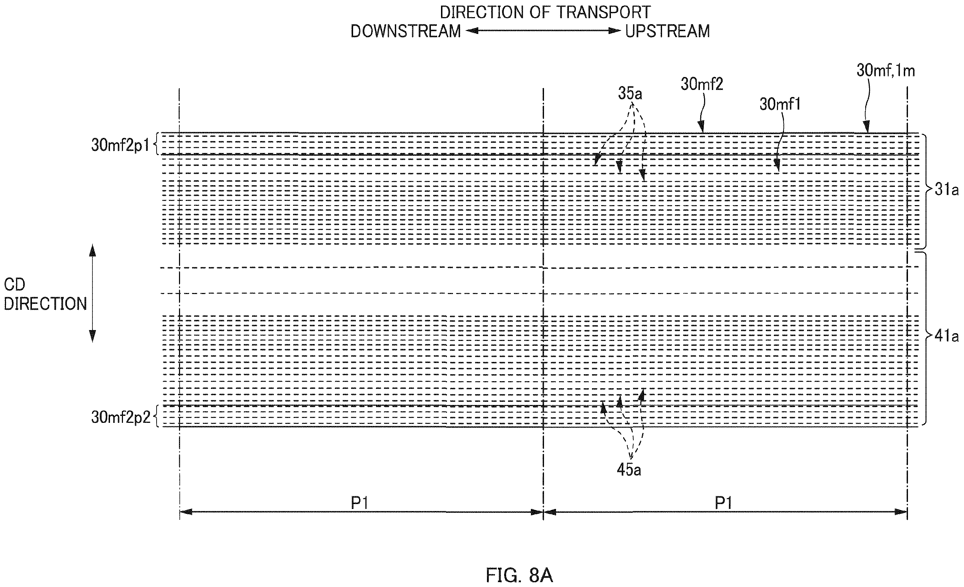

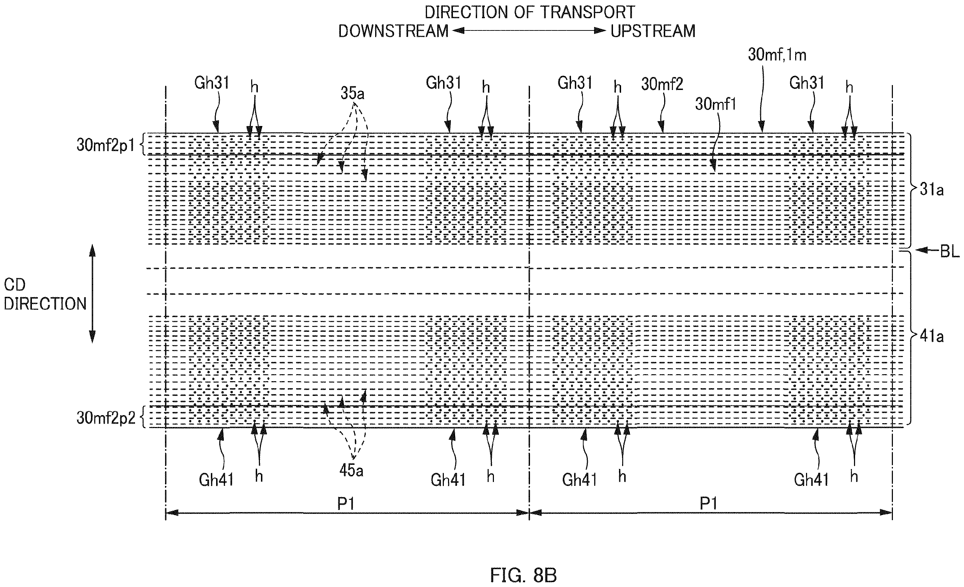

FIG. 8A is a schematic plan view of the intermediate product 1m immediately before being transported to the air-hole forming step S50 position, and FIG. 8B is a schematic plan view of the intermediate product 1m immediately after passing through the air-hole forming step S50.

As can be understood from a comparison of FIGS. 8A and 8B, in this air-hole forming step S50, the previously mentioned air holes h, h... (corresponding to hole portions) are formed in the intermediate product 1m, which is the base material for both the front band member 31 and the back band member 41. Here, in this manufacturing line, the intermediate product 1m is transported using so-called side flow transporting. Specifically, the intermediate product 1m is transported in an orientation in which the lateral direction of the diaper 1 conforms to the direction of transport, and the longitudinal direction conforms to the CD direction. For this reason, as show in FIG. 8A, when sent from an upstream step to the air-hole forming step S50 position, the intermediate product 1m is substantially constituted by one sheet-like member 30mf, in which a front-band-member continuous body 31a, which has multiple front band members 31 that are connected in the lateral direction, and a back-band-member continuous body 41a, which has multiple back band members 41 that are connected in the lateral direction, are connected in the CD direction as a single body.

More specifically, this sheet-like member 30mf has a continuous nonwoven fabric sheet 30mf1 (corresponding to a first nonwoven fabric sheet) and a continuous nonwoven fabric sheet 30mf2 (corresponding to a second nonwoven fabric sheet). The continuous nonwoven fabric sheet 30mf1 is continuous in the direction of transport, and has a size in the CD direction that corresponds to the sum value of the longitudinal dimension of the nonwoven fabric sheet 32 of the front band member 31 and the longitudinal dimension of the nonwoven fabric sheet 42 of the back band member 41. The continuous nonwoven fabric sheet 30mf2 is continuous in the direction of transport, and has a size in the CD direction that corresponds to the sum value of the longitudinal dimension of the nonwoven fabric sheet 33 of the front band member 31 and the longitudinal dimension of the nonwoven fabric sheet 43 of the back band member 41. These continuous nonwoven fabric sheets 30mf1 and 30mf2 are overlaid on each other so as to be overlapped in the thickness direction and joined to each other using a hot-melt adhesive. Also, elastic-string continuous bodies 35a and 45a, which are to become the previously mentioned elastic strings 35 and 45, are inserted between the continuous nonwoven fabric sheets 30mf1 and 30mf2 side-by-side in the CD direction in a state of being stretched in the direction of transport and continuous in the direction of transport, and are fixed to the continuous nonwoven fabric sheets 30mf1 and 30mf2. Furthermore, out of the two continuous nonwoven fabric sheets 30mf1 and 30mf2, the one continuous nonwoven fabric sheet 30mf1 has a larger CD direction size than the other continuous nonwoven fabric sheet 30mf2, and therefore the one continuous nonwoven fabric sheet 30mf1 has portions 30mf2p1 and 30mf2p2 that project out from the other continuous nonwoven fabric sheet 30mf2 on the two sides in the CD direction. These projecting portions 30mf2p1 and 30mf2p2 are folded inward in the CD direction, thus forming portions that correspond to the previously mentioned folded portions 33B and 43B.

Then, in the air-hole forming step S50, a pair of air hole groups Gh31 pertaining to the front band member 31 are repeatedly formed in the intermediate product 1m shown in FIG. 8A, which is the sheet-like member 30mf (corresponding to a composite sheet), in a region thereof on one side in the CD direction as shown in FIG. 8B at a product pitch P1 in the direction of transport. Also, a pair of air hole groups Gh41 pertaining to the back band member 41 are formed in a region on the other side at the product pitch P1 in the direction of transport. Note that the product pitch P1 mentioned here is substantially the same value as overall lateral lengths L31 and L41 of the front band member 31 and the back band member 41 in the unfolded state shown in FIG. 3.

Next, in the slitting step S60, the sheet-like member 30mf serving as the intermediate product 1m and having air holes h formed therein is folded in two in the CD direction as shown in FIG. 7, thus forming the front-band-member continuous body 31a and the back-band-member continuous body 41; the front-band-member continuous body 31a includes the elastic-string continuous bodies 35a in the uncut state, and the back-band-member continuous body 41a includes the elastic-string continuous bodies 45a in the uncut state.

In the subsequent gap forming step S70, the front-band-member continuous body 31a and the back-band-member continuous body 41a, which form the intermediate product 1m, are each moved outward in the CD direction as shown in FIG. 7. Accordingly, a gap is formed in the CD direction between the continuous bodies 31a and 41a, and the size of this gap corresponds to a longitudinal gap Ld between the front band member 31 and the back band member 41 in the diaper 1 in the unfolded state shown in FIG. 3.

In the subsequent front-elastic-string cutting step S80, as the front-band-member continuous body 31a passes, the elastic-string continuous bodies 35a are cut in a region AL1 that corresponds to the non-stretchy region AL in the continuous body 31a, thus forming the non-stretchy region AL in the front-band-member continuous body 31a. This will be described in detail below.

First, in this front-band-member continuous body 31a, as shown in the schematic plan view in FIG. 9A, the region on one side in the CD direction corresponds to the previously mentioned upper region AU in the front band member 31, and the region on the other side likewise corresponds to the lower region AD. At this point in time, in both of these regions, the elastic-string continuous bodies 35a extend in the direction of transport and are stretched in the direction of transport. It should be noted that in the region on the one side that corresponds to the upper region AU, the elastic-string continuous bodies 35a are fixed with a hot-melt adhesive over the entire length in the direction of transport, but in the region on the other side that corresponds to the lower region AD, there are fixed regions AH1, in which the elastic-string continuous bodies 35a are fixed, and a non-fixed region AL1, in which the elastic-string continuous bodies 35a are not fixed. The fixed regions and non-fixed region are side-by-side in the direction of transport. The non-fixed region AL1 is the same size as the previously mentioned non-stretchy region AL in the direction of transport, and is arranged at the product pitch P1 in the direction of transport.

Accordingly, as shown in the right portion in FIG. 9B, when the elastic-string continuous bodies 35a are cut at a predetermined position PC in the non-fixed region AL1, downstream end portions 35aed of the elastic-string continuous bodies 35a are cut away from the continuous bodies 35a. Accordingly, upstream portions 35eu of the cut-away elastic strings 35 contract toward the fixed region AH1 that is located on the downstream side. Also, due to this cutting, portions 35aedn of the elastic-string continuous bodies 35a, which become new downstream end portions thereof, contract toward the fixed region AH1 that is located on the upstream side. Accordingly, as shown in the left portion in FIG. 9B, the non-fixed region AL1 becomes a region in which the elastic strings 35 do not exist, and therefore the non-fixed region AL1 forms the previously mentioned non-stretchy region AL. On the other hand, as shown in FIG. 9B, in the fixed regions AH1, the cut-away elastic strings 35 and the newly-become-downstream end portions 35aedn remain due to being fixed with a hot-melt adhesive, and therefore these elastic strings 35 give stretchability to the fixed regions AH1. Accordingly, the fixed regions AH1 form the previously mentioned stretchy regions AH.

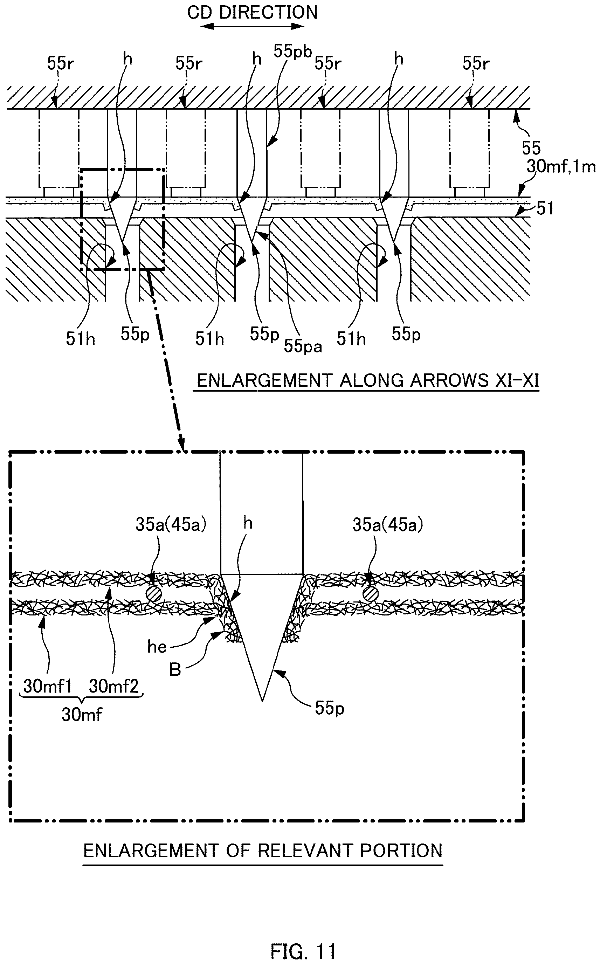

Note that here, concerning the hot-melt adhesive serving as the fixing portions, which are for fixing the elastic-string continuous bodies 35a in the stretchy regions AH corresponding to the fixed regions AH1, it is desirable that, the hot-melt adhesive is not applied to the continuous nonwoven fabric sheets 30mf1 and 30mf2 of the front-band-member continuous body 31a, but rather is applied to the elastic-string continuous bodies 35a. In addition, it is desirable that no adhesive other than the hot-melt adhesive is applied in the fixed regions AH1. Also, in this case, in the stretchy regions AH corresponding to the fixed regions AH1, the continuous nonwoven fabric sheets 30mf1 and 30mf2 are fixed to each other with use of merely the hot-melt adhesive applied to the elastic-string continuous bodies 35a and the previously described engagement of the continuous nonwoven fabric sheets 30mf1 and 30mf2 with each other in the edge portions he of the air holes h (enlarged view of a relevant portion in FIG. 11). Accordingly, in the stretchy regions AH of the front-band-member continuous body 31a, besides the adhesive and the edge portions he, fixing portions for fixing the continuous nonwoven fabric sheets 30mf1 and 30mf2 to each other are not provided. Specifically, in these stretchy regions AH, fixing portions solely for fixing the continuous nonwoven fabric sheets 30mf1 and 30mf2 to each other are not provided. Accordingly, it is possible to improve the softness of the front-band-member continuous body 31a. Also, in this example, in the edge portions he of the air holes h, the continuous nonwoven fabric sheets 30mf1 and 30mf2 are fixed to each other by merely engagement of the constituent fibers of the continuous nonwoven fabric sheets 30mf1 and 30mf2 with each other, but in some cases, an adhesive is provided in the edge portions he, and this adhesive may contribute to the fixing of the nonwoven fabric sheets 30mf1 and 30mf2 to each other. It should be noted that from the viewpoint of softening the front-band-member continuous body 31a, it is desirable that an adhesive is not provided in the edge portions he of the air holes h.

In the subsequent back-elastic-string cutting step S90, as the back-band-member continuous body 41a shown in FIG. 7 passes, the elastic-string continuous bodies 45a are cut in a region AL1 that corresponds to the non-stretchy region AL in the continuous body 41a, thus forming the non-stretchy region AL in the back-band-member continuous body 41a. Note that the processing for forming the non-stretchy region AL through this cutting is substantially the same as the above-described cutting performed on the front-band-member continuous body 31a. A description therefore will not be given for this.

In the subsequent absorbent-main-body attaching step S100, the cutform-shaped absorbent main body 10, which is produced using other steps that are not shown, is fixed in a manner of spanning between the two band member continuous bodies 31a and 41a that are sent as the intermediate product 1m from the elastic string cutting steps S80 and S90. Accordingly, the intermediate product 1m becomes an approximately ladder-shaped diaper continuous body 1Ha that is formed by a series of diapers 1H that are unfolded and approximately H-shaped as shown in FIG. 3.

In the subsequent folding step S110, the approximately ladder-shaped diaper continuous body 1Ha, which is the intermediate product 1m, is folded in two such that the front-band-member continuous body 31a and the back-band-member continuous body 41a are overlaid on one another in the thickness direction.

In the subsequent side sealing step S120, the front-band-member continuous body 31a and the back-band-member continuous body 41a, which were overlaid on one another in the folding step S110, are welded at positions corresponding to the lateral end portions 31e and 41e of the diaper 1 to form side seal portions SS, thus being fixed in a state of being folded in two. As a result, the intermediate product 1m becomes the pull-on diaper continuous body 1a in which multiple pull-on diapers 1 are connected in the lateral direction.

Then, in the final cutting step S130, the pull-on diaper continuous body 1a, which is the intermediate product 1m, is cut at each of the side seal portions SS, and individual pull-on diapers 1 are thus manufactured.

The following describes the steps S50 to S100 in further detail, but the steps from the folding step S110 to step S130 will not be described any further because it is clear that they can be appropriately realized using a known method.

### Air-hole forming step S50

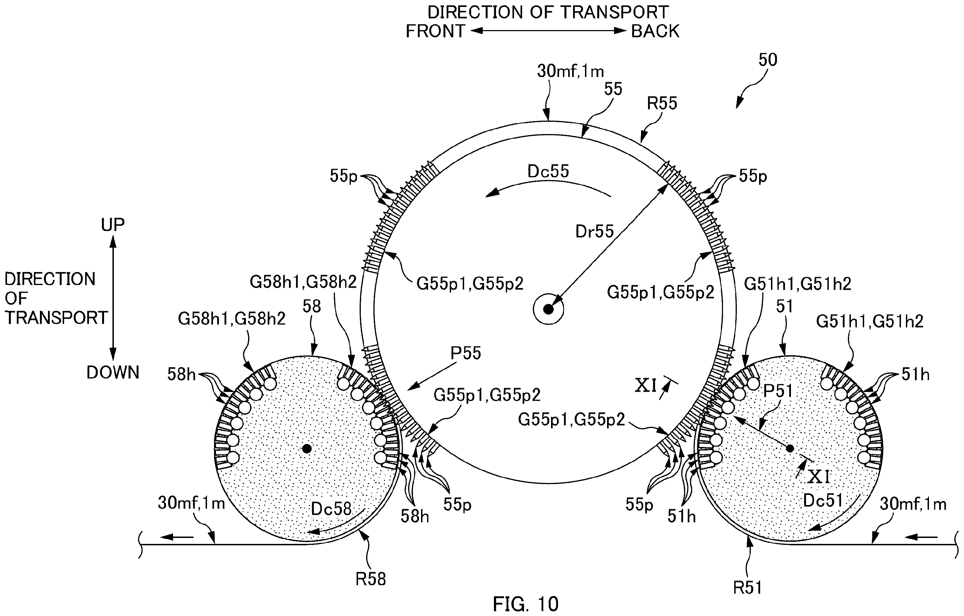

In the air-hole forming step S50, an air-hole forming device 50 (corresponding to a hole-portion forming device) is provided for forming the air holes h in the sheet-like member 30mf. FIG. 10 is a schematic side view of the device 50, in which portions of the configuration (upstream roller 51 and downstream roller 58) are shown in a longitudinal cross-sectional view, and FIG. 11 is a schematic side view taken along arrows XI-XI in FIG. 10.

As shown in FIG. 10, the air-hole forming device 50 has three rollers 51, 55, and 58 that are driven to rotate about rotation shafts that extend in the CD direction. Specifically, the rollers 51, 55, and 58 are arranged side-by-side as an upstream roller 51, an intermediate roller 55, and a downstream roller 58 in this order from upstream to downstream in the direction of transport. Also, the intermediate roller 55 is arranged adjacent to the upstream roller 51 and the downstream roller 58 such that the outer circumferential surfaces thereof face each other. The above-described sheet-like member 30mf, which is sent as the intermediate product 1m to the air-hole forming device 50, is transported along an approximately Ω-shaped transporting route due to the rotation operations of these three rollers 51, 55, and 58. Specifically, the sheet-like member 30mf is first transported along an arc-shaped first transporting route R51 for becoming wrapped around the upstream roller 51, then transported along an arc-shaped second transporting route R55 for becoming wrapped around the intermediate roller 55, and finally transported along an arc-shaped third transporting route R58 for becoming wrapped around the downstream roller 58. Thereafter, the sheet-like member 30mf separates from the downstream roller 58 and is sent to the subsequent slitting step S60.

Note that the upstream roller 51 and the intermediate roller 55 are closest to each other at a predetermined position P51 in a rotation direction Dc51 of the upstream roller 51, and this closest position P51 is the position of switching from the first transporting route R51 to the second transporting route R55. Similarly, the intermediate roller 55 and the downstream roller 58 are closest to each other at a predetermined position P55 in a rotation direction Dc55 of the intermediate roller 55, and this closest position P55 is the position of switching from the second transporting route R55 to the third transporting route R58.

The intermediate roller 55 has multiple pin members 55p, 55p... (press-in members) that project from the outer circumferential surface. The pin members 55p, 55p... are members that have a tapered shape on the tip side. Specifically, as shown in FIG. 11, in this example, the pin members 55p each include a conical portion 55pa on the tip side, and a circular column portion 55pb, which has the same diameter as the bottom face of the conical portion 55pa, on the base side, and these portions are integrated with each other. Also, hole portions 51h, 51h... capable of receiving insertion of the pin members 55p, 55p... are provided in the outer circumferential surface of the upstream roller 51. Specifically, the hole portions 51h have a larger diameter than the conical portions 55pa of the pin members 55p in the range of insertion of the pin members 55p. Also, the hole portions 51h are formed so as to correspond to the pin members 55p, such that only one pin member 55p is inserted into each hole portion 51h at the above-described closest position P51.

Accordingly, when the formation target portion, where the air holes h are to be formed, of the sheet-like member 30mf located on the upstream roller 51 shown in FIG. 10 passes the closest position P51, corresponding pin members 55p are inserted into the hole portions 51h of the upstream roller 51 as shown in FIG. 11. Therefore, the pin members 55p are smoothly pressed into the formation target portion of the sheet-like member 30mf, thus swiftly forming the air holes h in the formation target portion.

It should be noted that in this example, given that the air holes h are formed at the closest position P51 as described above, the closest position P51 corresponds to a hole-portion formation position according to the Claims.

Also, as shown in the enlarged view of a relevant portion in FIG. 11, when the air holes h are formed, the continuous nonwoven fabric sheets 30mf1 and 30mf2 that constitute the sheet-like member 30mf are engaged with each other in the edge portions he of the air holes h. Specifically, the burrs B that project in the direction of press-in the pin member 55p are formed in the edge portions he, and the constituent fibers of the continuous nonwoven fabric sheet 30mf1 and the constituent fibers of the continuous nonwoven fabric sheet 30mf2 are engaged with each other through entanglement or the like in the burrs B and the vicinity thereof. For this reason, it can also be said that the continuous nonwoven fabric sheets 30mf1 and 30mf2 are joined to each other by the edge portions he of the air holes h. Due to the engagement of the continuous nonwoven fabric sheets 30mf1 and 30mf2 with each other in these edge portions he, the elastic-string continuous bodies 35a (45a) between the continuous nonwoven fabric sheets 30mf1 and 30mf12 are prevented from intruding into the air holes h, and as a result, it is possible to prevent the problem that the elastic strings 35 (45) are visible through the air holes h in the diaper 1 that is ultimately manufactured.

Also, the apex angle of the conical portion 55pa in FIG. 11 is selected from the range of 20° to 45° for example, and is 36° in this example. Also, the height of the conical portion 55pa is selected from the range of 3 mm to 8 mm for example, and is 4.6 mm in this example. Note that there is no limitation whatsoever to this. Furthermore, although the edge portions of the hole portions 51h are chamfered in this example, there is no limitation whatsoever to this, or in other words, the edge portions are not required to be chamfered.

Also, in this example, the intermediate roller 55 in FIG. 10 is a large-diameter roller having a circumferential length that corresponds to approximately two times the above-described product pitch P1, such that two pairs of air hole groups Gh31 and two pairs of air hole groups Gh41 corresponding to two diapers shown in FIG. 8B can be formed over one full rotation. Also, the upstream roller 51 in FIG. 10 is a small-diameter roller having a circumferential length that substantially corresponds to the product pitch such that one pair of air hole groups Gh31 and one pair of air hole groups Gh41 corresponding to one diaper shown in FIG. 8B can be formed over one full rotation. Note that there is no limitation whatsoever to this.

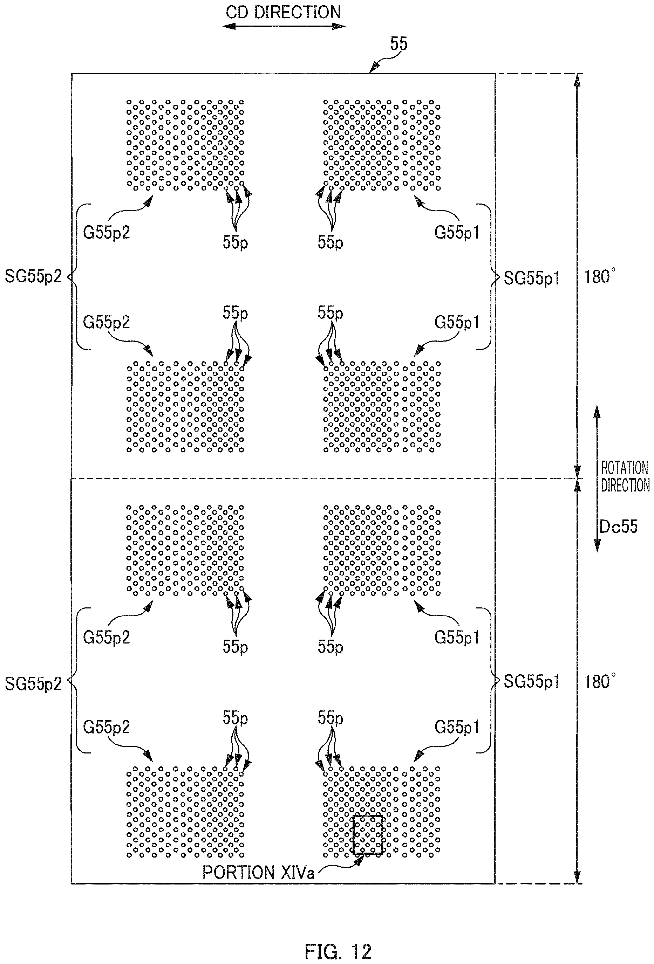

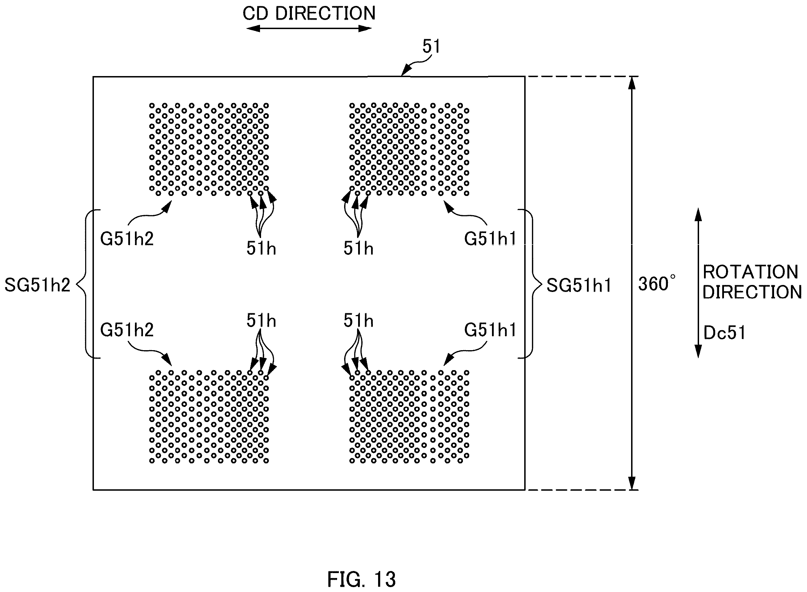

FIG. 12 is a diagram illustrating an arrangement pattern of the pin members 55p on the outer circumferential surface of the intermediate roller 55, and FIG. 13 is a diagram illustrating an arrangement pattern of the hole portions 51h on the outer circumferential surface of the upstream roller 51. Note that these diagrams both show the outer circumferential surfaces of the rollers 55 and 51 in a state of being spread flat. Also, later-described receiving portions 55r are not shown in FIG. 12 in order to prevent complexity in the drawing.

As shown in FIG. 12, the pin members 55p are provided in correspondence with the air hole groups Gh31 and Gh41 that are formed in the previously described sheet-like member 30mf. Specifically, as shown in FIG. 8B, in the region of the sheet-like member 30mf that is on the one side in the CD direction, one pair of air hole groups Gh31 for the front band member 31 is to be formed in each diaper, and therefore, as shown in FIG. 12, in the region of the outer circumferential surface of the intermediate roller 55 that is on the one side in the CD direction, a pair of pin member groups G55p1 are provided side-by-side in the rotation direction Dc55, each having pin members 55p in the same staggered arrangement as the air hole groups Gh31. Similarly, in the region of the sheet-like member 30mf that is on the other side in the CD direction, one pair of air hole groups Gh41 for the back band member 41 is to be formed in each diaper, and therefore, in the region of the outer circumferential surface of the intermediate roller 55 that is on the other side in the CD direction, a pair of pin member groups G55p2 are provided side-by-side in the rotation direction Dc55, each having pin members 55p in the same staggered arrangement as the air hole groups Gh41.

Also, in this example, as previously described, the intermediate roller 55 is provided with air holes h corresponding to two diapers for each full rotation. Accordingly, letting one pin member group set SG55p1 (SG55p2) be one set of two pin member groups G55p1, G55p1 (G55p2, G55p2) that are side-by-side in the rotation direction Dc55, the outer circumferential surface of the intermediate roller 55 is provided with two pin member group sets SG55p1, SG55p1 (SG55p2, SG55p2) that are side-by-side at an even pitch of 180° in the direction of rotation Dc55.

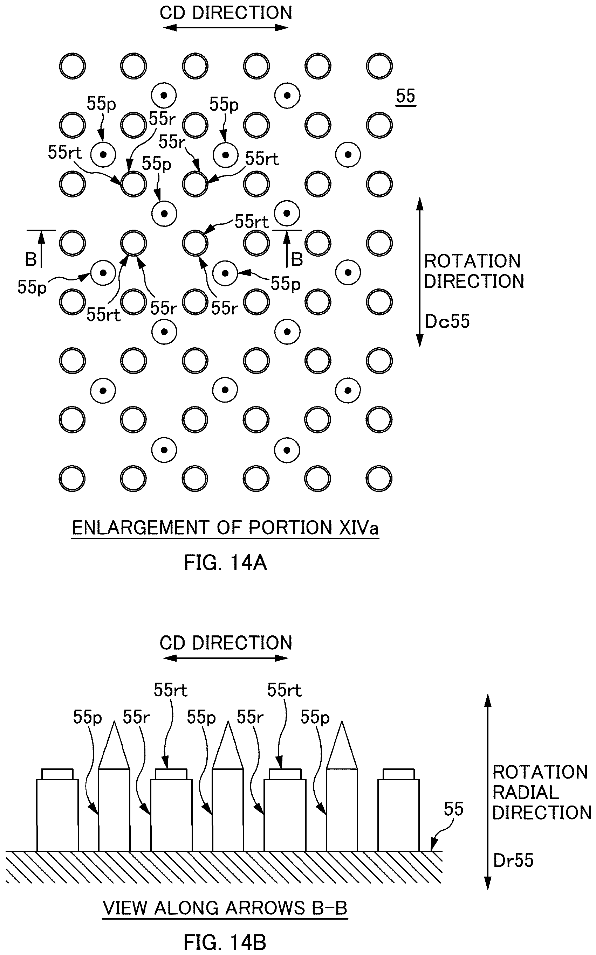

Furthermore, FIG. 14A shows an enlarged view of a portion XIVa in FIG. 12, and FIG. 14B shows a view taken along arrows B-B in FIG. 14A. As shown in FIG. 14A, receiving portions 55r that are separate from the pin members 55p are formed in the outer circumferential surface of the intermediate roller 55 as projections at positions between adjacent pin members 55p. Also, as shown in FIG. 14B, the receiving portions 55r are each an approximately circular columnar body that has, at the leading end, a top surface 55rt that faces outward in a rotation radial direction Dr55 of the intermediate roller 55. Accordingly, the sheet-like member 30mf is received and supported by not only the pin members 55p, but also the receiving portions 55r. For this reason, the sheet-like member 30mf can overall be stably supported at an appropriate position in the rotation radial direction Dr55 of the intermediate roller 55. Also, at this time, the top surfaces 55rt of the receiving portions 55r receive the one surface of the sheet-like member 30mf without penetrating it, and thus also effectively contributes to stably supporting the sheet-like member 30mf at the appropriate position.

Also, in this example, as shown in FIG. 14A, the receiving portions 55r are provided at four locations around each pin member 55p, thus making it possible to reliably receive the sheet-like member 30mf, but there is no limitation whatsoever to the aforementioned four locations. For example, if arrangement space cannot be ensured, the number of locations may be smaller, or on the other hand, if arrangement space can be ensured, the number of locations may be larger.

Furthermore, in the example shown in FIG. 14B, the positions of the top surfaces 55rt of the receiving portions 55r are inward in the rotation radial direction Dr55 of the intermediate roller 55 relative to the positions of the apexes of the pin members 55p. This therefore prevents the receiving portions 55r from hindering the processing for forming the air holes h with use of the pin members 55p. Note that here, it is desirable that the top surface 55rt of the receiving portion 55r is located in the vicinity of the bottom face of the conical portion 55pa of the pin member 55p, and for example, it is desirable that the position of the top surface 55rt in the rotation radial direction Dr55 is in a range of ±2 mm centered about the position of the bottom face. In this example, the top surface 55rt is matched with the bottom face of the conical portion 55pa. Accordingly, the receiving portions 55r can reliably receive the sheet-like member 30mf without hindering the processing for forming the air holes h with use of the pin member 55p. Accordingly, the pin members 55p can swiftly form the air holes h in the sheet-like member 30mf.

Also, in this example, the top surface 55rt of the receiving portion 55r is shaped as a perfect circle having a diameter selected from the range of 2 mm to 5 mm for example, but there is no limitation whatsoever to this. For example, it may be shaped as a polygon such as an equilateral triangle or a square, or may have another shape.

Also, as shown in FIG. 13, the hole portions 51h in the outer circumferential surface of the upstream roller 51 are provided in correspondence with the pin members 55p. Specifically, as shown in FIG. 12, the two pin member groups G55p1 are provided side-by-side in the rotation direction Dc55 in the region of the outer circumferential surface of the intermediate roller 55 that is on the one side in the CD direction, and therefore in correspondence with this, two hole portion groups G51h1, each of which has multiple hole portions 51h in a staggered arrangement, are provided side-by-side in the rotation direction Dc51 in the region of the outer circumferential surface of the upstream roller 51 that is on the one side in the CD direction. Similarly, as shown in FIG. 12, the two pin member groups G55p2 are provided side-by-side in the rotation direction Dc55 in the region of the outer circumferential surface of the intermediate roller 55 that is on the other side in the CD direction, and therefore in correspondence with this, two hole portion groups G51h2, each of which has multiple hole portions 51h in a staggered arrangement, are provided side-by-side in the rotation direction Dc51 in the region of the outer circumferential surface of the upstream roller 51 that is on the other side in the CD direction.

It should be noted that as previously described, the upstream roller 51 contributes to forming air holes h corresponding to one diaper for each full rotation, and therefore letting one hole portion group set SG51h1 (SG51h2) be one set of two hole portion groups G51h1, G51h1 (G51h2, G51h2) that are side-by-side in the rotation direction Dc51, the outer circumferential surface of the upstream roller 51 is provided with only one hole portion group set SG51h1 (SG51h2).

Also, in some cases, a heating device (not shown) such as an electrothermal heater is provided in the intermediate roller 55 in FIG. 10, and the pin members 55p of the intermediate roller 55 may be heated by it. According to this configuration, the thermoplastic resin constituent fibers of the continuous nonwoven fabric sheets 30mf1 and 30mf2 of the sheet-like member 30mf can be softened with use of the heated pin members 55p. Accordingly, the pin members 55p can easily push the constituent fibers to the side when being pressed into the sheet-like member 30mf, thus more easily forming the air holes h. Also, by raising the heating temperature, the edge portions he of the air holes h can be melted partially or over the entire circumference, and therefore the continuous nonwoven fabric sheets 30mf1 and 30mf2 of the sheet-like member 30mf are welded to each other in the edge portions he. Accordingly, the continuous nonwoven fabric sheets 30mf1 and 30mf2 can be engaged with each other more strongly in the previously described edge portions he.

It should be noted that one example of a standard for heating is that the temperature of the pin members 55p is greater than or equal to the softening point of the thermoplastic resin, and in some cases is greater than or equal to the melting point thereof. The softening point can be obtained by TMA (thermomechanical analysis) in accordance with JIS K 7196 (Testing Method for Softening temperature of Thermoplastic Film and sheeting by Thermomechanical Analysis). Also, the melting point can be obtained as the melting peak temperature in DSC (differential scanning calorimetry) in accordance with JIS K 7121 (Testing Methods for Transition temperatures of Plastics).

Also, the configuration of the downstream roller 58 in FIG. 10 is substantially the same as that of the upstream roller 51. For example, the outer circumferential surface of the downstream roller 58 is provided with hole portion groups G58h1 and G58h2 that have the same specifications as the hole portion groups G51h1 and G51h2 in the outer circumferential surface of the upstream roller 51. Accordingly, when the sheet-like member 30mf is received from the intermediate roller 55, the pin members 55p of the intermediate roller 55 are smoothly inserted into hole portions 58h in the hole portion groups G58h1 and G58h2, thus making it possible to receive the sheet-like member 30mf without largely deforming the shape of the air holes h formed in the sheet-like member 30mf.

### Slitting step S60

As shown in FIG. 7, in the slitting step S60, the sheet-like member 30mf having air holes h formed therein, which is sent from the air-hole forming step S50 located upstream thereof, is divided into two portions at a boundary position BL (FIG. 8B) between the regions on the one side and the other side in the CD direction. Accordingly, the front-band-member continuous body 31a, in which the elastic-string continuous bodies 35a are uncut, and the back-band-member continuous body 41a, in which the elastic-string continuous bodies 45a are uncut, are produced side-by-side in the CD direction (FIG. 7).

This dividing processing can be carried out using a known slitter device 60 shown in FIG. 7. Specifically, for example, this device 60 has a pair of upper and lower disc-shaped rotary blades, and divides the sheet-like member 30mf into two portions in the CD direction using the blade edges at the peripheral edges of the rotating blades. This type of device 60 is well-known. Accordingly, this will not be described further.

### Gap forming step S70

As shown in FIG. 7, in the gap forming step S70, the front-band-member continuous body 31a and the back-band-member continuous body 41a, which are sent from the slitting step S60 located upstream thereof, are each moved outward in the CD direction, thus forming a gap between the continuous bodies 31a and 41a in the CD direction, and this gap has a size corresponding to the longitudinal gap Ld between the front band member 31 and the back band member 41 in the diaper 1 in the unfolded state in FIG. 3.

Note that the movement of the front-band-member continuous body 31a outward in the CD direction and the movement of the back-band-member continuous body 41a outward in the CD direction can be realized by arranging multiple transporting rollers (not shown) along the transporting route in consideration of such movement, and the detail of such a configuration can be arrived at based on the above description. Accordingly, this will not be described further.

### Front elastic string cutting step S80

In the front-elastic-string cutting step S80, the front-band-member continuous body 31a is sent from the gap forming step S70 located upstream thereof and is shown in FIG. 9A. As shown in FIG. 9B, in the front-band-member continuous body 31a, the elastic-string continuous bodies 35a are cut in the non-fixed region AL1 that corresponds to the non-stretchy region AL, and the elastic-string continuous bodies 35a are not cut in the fixed regions AH1 that correspond to the stretchy regions AH. Accordingly, the non-stretchy region AL and the stretchy regions AH are formed in the front-band-member continuous body 31a. Note that the region AL1 corresponding to the non-stretchy region AL appears at the product pitch P1 of the diaper 1 in the direction of transport. Accordingly, this cutting is performed on the front-band-member continuous body 31a at the product pitch P1 of the diaper 1.

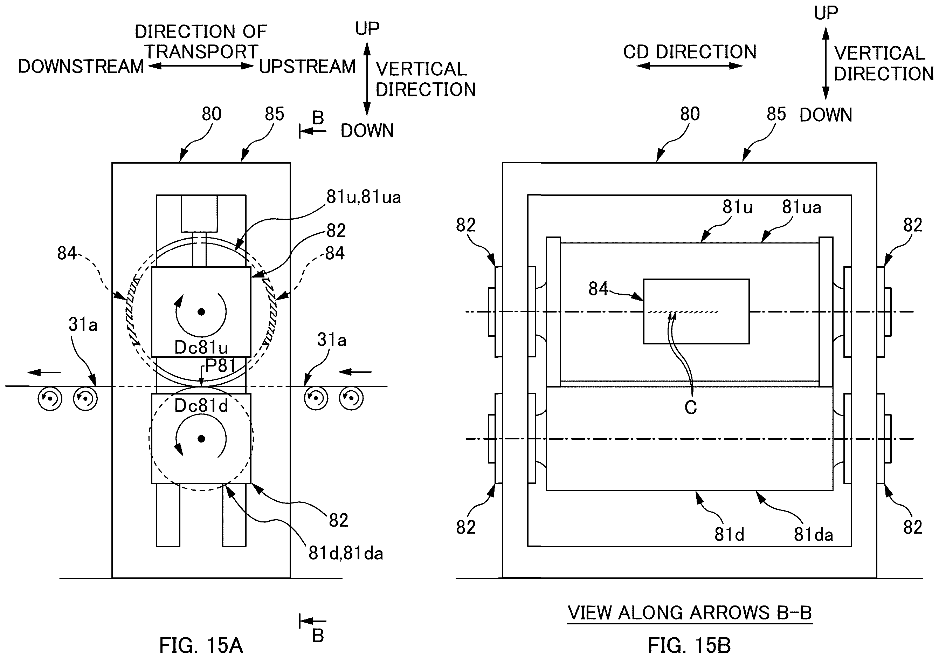

This cutting is performed by a front-side cutter device 80 (corresponding to an elastic-member cutting device) . FIG. 15A is a schematic side view of this cutter device 80, and FIG. 15B is a view taken along arrows B-B in FIG. 15A.

As shown in FIG. 15A, the cutter device 80 has a pair of upper and lower rolls 81u and 81d that are driven to rotate about rotation shafts that extend in the CD direction and that respectively have outer circumferential surfaces 81ua and 81da that face each other, and a housing 85 that supports these rolls rolls 81u and 81d via bearing members 82.

The rolls 81u and 81d are rotated in rotation directions Dc81u and Dc81d about rotation shafts by servomotors (not shown) that serve as drive sources. Accordingly, when the front-band-member continuous body 31a is fed to a position between the outer circumferential surfaces 81ua and 81da of the pair of upper and lower rolls 81u and 81d, the front-band-member continuous body 31a passes between the outer circumferential surfaces 81ua and 81da and is fed downstream in the direction of transport.

Also, in this example, the upper roll 81u is a cutter roll, and the lower roll 81d is an anvil roll. The outer circumferential surface 81ua of the upper roll 81u is provided with at least one cutter block 84 at an angle in the rotation direction Dc81u that corresponds to the product pitch P1 of the diaper 1. Specifically, in this example, as shown by hatching in FIG. 15A, two cutter blocks 84 are provided at intervals of 180° in the rotation direction Dc81u. Multiple cutter blades C project from each of the cutter blocks 84, and the outer circumferential surface 81da of the lower roll, which is the anvil roll, is a smooth surface for receiving the cutter blades C, C.... Also, these rolls 81u and 81d are controlled to rotate in coordination with the operation for transporting the front-band-member continuous body 31a, and therefore the cutter roll 81u rotates such that each time the previously described non-fixed region AL1 corresponding to the non-stretchy region AL passes, the cutter block 84 faces the non-fixed region AL1.

Accordingly, each time the non-fixed region AL1, which corresponds to the non-stretchy region AL in FIG. 9A, passes a closest position P81 (corresponding to a cutting position), which is located between the outer circumferential surfaces 81ua and 81da of the pair of rolls 81u and 81d and is the position at which the outer circumferential surfaces 81ua and 81da are closest to each other, the non-fixed region AL1 is pressed between the cutter blades C, C... of one of the cutter blocks 84 of the cutter roll 81u and the outer circumferential surface 81da of the anvil roll 81d. The elastic-string continuous bodies 35a, 35a... are thus cut at the pressed positions PC.

Due to this cutting, as shown in the right portion of FIG. 9B, the downstream end portions 35aed of the elastic-string continuous bodies 35a are cut away from the continuous bodies 35a, and the upstream portions 35eu of the cut-away elastic strings 35 contract toward the fixed region AH1 that is located on the downstream side. Also, due to this cutting, the portions 35aedn of the elastic-string continuous bodies 35a, which become new downstream end portions thereof, contract toward the fixed region AH1 that is located on the upstream side. Accordingly, as shown in the left portion in FIG. 9B, the non-fixed region AL1 becomes a region in which the elastic strings 35 do not exist, and therefore the non-fixed region AL1 forms the previously mentioned non-stretchy region AL. On the other hand, as shown in FIG. 9B, in the fixed regions AH1, the portions 35aedn, which are the new downstream end portions of the cut-away elastic strings 35, remain due to being fixed with a hot-melt adhesive, and therefore these elastic strings 35 give stretchability to the fixed regions AH1. Accordingly, the fixed regions AH1 form the previously mentioned stretchy regions AH.

Also, as shown in FIG. 9A, before this cutting, the previously described air holes h are already formed in the fixed region AH1 that corresponds to the stretchy region AH in the front-band-member continuous body 31a, and as previously described, the continuous nonwoven fabric sheets 30mf1 and 30mf2 that constitute the front-band-member continuous body 31a are engaged with each other in the edge portions he of the air holes h due to entangling of the constituent fibers or the like (see the enlarged view of a relevant portion in FIG. 11). For this reason, even if low fixing strength of the elastic-string continuous bodies 35a and the elastic strings 35 in the fixed region AH1 allows the cut and contracting portions 35eu and 35aedn of the elastic string to intrude a large amount into the fixed region AH1, and the portions 35eu and 35aedn deviate in the CD direction and attempt to move toward the air holes h, further movement toward the air holes h is suppressed because the continuous nonwoven fabric sheets 30mf1 and 30mf2 are engaged with each other in the edge portions he of the air holes h in the enlarged view of a relevant portion in FIG. 11. Accordingly, it is possible to prevent a state where the elastic strings 35 can be seen through the air holes h after contracting of the elastic strings 35 and the elastic-string continuous bodies 35a, and this consequently enables achieving a favorable appearance for the front-band-member continuous body 31a.

As shown in FIG. 15B, each of the cutter blocks 84 on the outer circumferential surface 81ua of the cutter roll 81u is provided with one cutter blade C for each of the elastic-string continuous bodies 35a, 35a... in this example. For example, 14 elastic-string continuous bodies 35a, 35a... are arranged in the lower region AD of the front-band-member continuous body 31a as shown in FIG. 9B, and therefore 14 cutter blades C, C... are provided in each of the cutter blocks 84. Accordingly, the cutter blades C each cut one corresponding elastic-string continuous body 35a at one location PC in the direction of transport.

Note that there is no limitation whatsoever to this. For example, in order to reliably cut each elastic-string continuous body 35a, multiple cutter blades C, C... may be provided in correspondence with each elastic-string continuous body 35a. Specifically, multiple cutter blades C, C... may be arranged side-by-side on a straight line extending in the rotation direction Dc81u of the cutter roll 81u.

Also, in this example, the blade tips of the cutter blades C, C... have a straight shape in a plan view. Also, the length LC of each blade tip is selected from the range of 4 mm to 15 mm for example, and the width WC of each blade tip is selected from the range of 0.1 mm to 0.5 mm for example. It should be noted that the shape of the blade tip is not limited whatsoever to being straight as described above, and may be shaped as a polygonal line having a zigzag shape, or may be shaped as a curved line having a circular arc shape.

Furthermore, a linear crimped portion or a linear cut portion is formed as a mark TC where the elastic-string continuous bodies 35a were cut at each position PC where the cutter blades C are pressed during the cutting (FIG. 9B). Note that the crimped portion is a portion where the continuous nonwoven fabric sheets 30mf1 and 30mf2 of the front-band-member continuous body 31a are crimped together, and a linear cut portion is a portion where the continuous nonwoven fabric sheets 30mf1 and 30mf2 are cut and pierced.

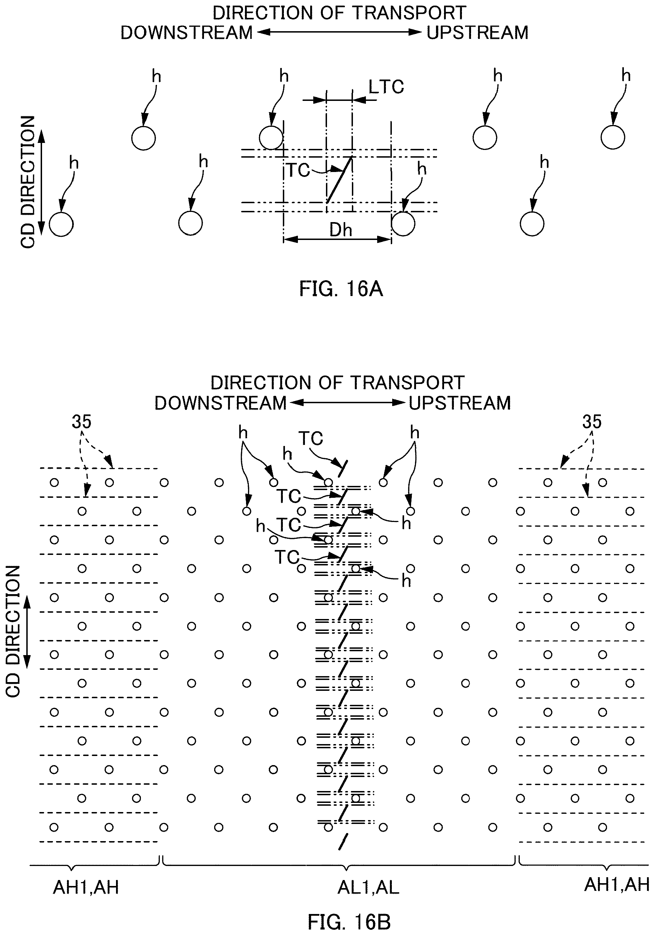

Here, it is desirable that, as shown in FIG. 16A, the cutting is performed such that each of the marks TC is not overlapped with, in the CD direction, the pair of air holes h that are located closest to the mark TC on the two sides in the direction of transport. According to this configuration, even if an elastic-string continuous body 35a needs to be cut at a position close to an air hole h, the elastic-string continuous body 35a can be cut without the shape of the air hole h being deformed by the cutter blade C. For example, even if the air holes h are formed in the non-fixed region AL1 as in the example shown in FIG. 16B, the elastic-string continuous bodies 35a can be cut without the shape of the air holes h being deformed by the cutter blades C.

It should be noted that in this example in FIG. 16B, all of the marks TC are in a positional relationship of not being overlapped with the air holes h. However, there is no limitation whatsoever to this. Specifically, even if at least one of the marks TC is in this non-overlapping positional relationship, the above-described actions and effects can be reasonably achieved. For this reason, a configuration is possible in which one mark TC satisfies the non-overlapping positional relationship. Also, this cutting can be realized by adjusting the relative positions of the pin members 55p and the cutter blades C in the CD direction such that the marks TC and the air holes h are in the above-described positional relationship.

Also, it is furthermore desirable that, as shown in FIG. 16A, a size LTC of each of the marks TC in the direction of transport is smaller than a distance Dh in the direction of transport between the pair of air holes h that are located closest to the mark TC on the two sides in the direction of transport. According to this configuration, it is possible to more reliably prevent the problem that the shape of the air holes h is deformed by the cutter blades C. Note that in the example in FIG. 16B, all of the marks TC are in the above-described size relationship with the corresponding pairs of air holes h. However, there is no limitation whatsoever to this. Specifically, even if at least one of the marks TC is in the above-described size relationship with the corresponding pair of air holes h, the above-described actions and effects can be reasonably achieved. For this reason, a configuration is possible in which one mark TC satisfies this size relationship. Also, this cutting can be realized by adjusting the relative positional relationship between each of the cutter blades C and the pair of pin members 55p located on the two sides thereof in the direction of transport such that the size of the blade tip of the cutter blade C in the direction of transport is smaller than the distance between the pair of pin members 55p in the direction of transport.

### Back elastic string cutting step S90

As shown in FIG. 7, in the back-elastic-string cutting step S90, cutting that is similar to the processing described with reference to FIGS. 9A and 9B is performed on the back-band-member continuous body 41a that is sent from the gap forming step S70 located upstream thereof. Specifically, the elastic-string continuous bodies 45a are cut in the region AL1 corresponding to the non-stretchy region AL, and the elastic-string continuous bodies 45a are not cut in the regions AH1 corresponding to the stretchy regions AH. Accordingly, the non-stretchy region AL and the stretchy regions AH are formed in the back-band-member continuous body 41a. Note that as can be understood from the above description, this cutting is substantially the same as the previously described front-elastic-string cutting step S80, and the configuration of a back-side cutter device 90 (corresponding to an elastic-member cutting device), which is shown in FIG. 7 and is for performing this cutting processing, is also the same as the front-side cutter device 80. Accordingly, a detailed description will not be given for it here.

### Absorbent-main-body attaching step S100

As shown in FIG.7, in the absorbent-main-body attaching step S100, the cutform-shaped absorbent main body 10, which is produced in other steps that are not shown, is arranged spanning between and fixed to the band member continuous bodies 31a and 41a that are received as the intermediate product 1m from the elastic string cutting steps S80 and S90 located upstream thereof, and therefore the intermediate product 1m becomes the approximately ladder-shaped diaper continuous body 1Ha that is formed by a series of diapers 1H that are unfolded and approximately H-shaped as shown in FIG. 3.

Here, at a time when the absorbent main body 10 is to be attached, the front-band-member continuous body 31a and the back-band-member continuous body 41a have already been provided with the air holes h, and the elastic-string continuous bodies 35a and 45a have already been cut. Accordingly, it is possible to prevent a problem that can occur in the case where processing for forming the air holes h and processing for cutting the elastic-string continuous bodies 35a and 45a are performed after the absorbent main body 10 is attached to the band member continuous bodies 31a and 41a for example, that is to say a problem that this hole forming and cutting are performed on the absorbent main body 10 as well, thus damaging the absorbent main body 10. It should be noted that this processing for attaching the absorbent main body 10 can be realized using, for example, a rotating drum (not shown) in which holding portions for suctioning and holding the absorbent main body 10 to the outer circumferential surface are provided side-by-side at the product pitch P1 in the rotation direction, and this rotating drum is well-known. Accordingly, a detailed description will not be given for it.

### Second Embodiment

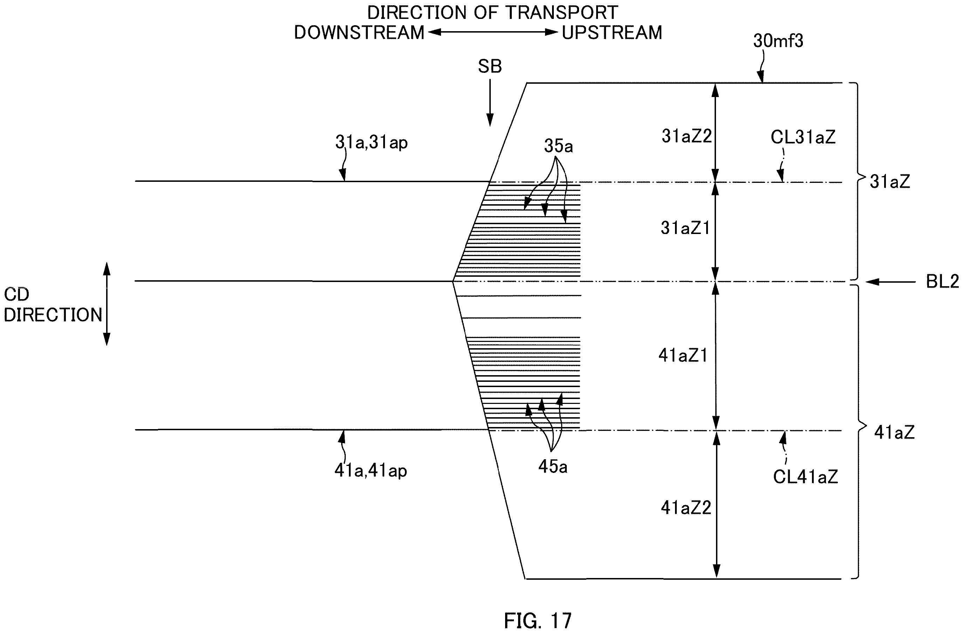

FIG. 17 is a diagram schematically illustrating a manufacturing method of a second embodiment.

In the first embodiment described above, as shown in FIG. 8A, the sheet-like member 30mf, which has a two-layer structure and is to be sent to the air-hole forming step S50, is formed by overlaying the two continuous nonwoven fabric sheets 30mf1 and 30mf2, which are separate from each other, in the thickness direction, but a main difference in the second embodiment is that, as shown in FIG. 17, the sheet-like member 30mf having a two-layer structure is formed from one continuous nonwoven fabric sheet 30mf3. Other aspects are approximately the same as in the first embodiment described above. Accordingly, the same or similar configurations are denoted by the same reference signs, and will not be described further.

As shown in FIG. 17, in the second embodiment, first, one base sheet 30mf3 is fed from an original fabric coil (not shown) as the material for the sheet-like member 30mf. This base sheet 30mf3 is, for example, a single-layer continuous nonwoven fabric sheet that is continuous in the direction of transport, which is a direction that intersects the CD direction. The base sheet 30mf3 has a front-side material portion 31aZ (corresponding to a third portion) that is to become the front-band-member continuous body 31a on one side in the CD direction, and has a back-side material portion 41aZ (corresponding to a third portion) that is to become the back-band-member continuous body 41a on the other side. Also, the front-side material portion 31aZ has a size corresponding to two front-band-member continuous bodies 31a in the CD direction, and the back-side material portion 41aZ has a size corresponding to two back-band-member continuous bodies 41a in the CD direction.

Accordingly, when passing through a folding step SB that is arranged at a predetermined position in the direction of transport, a known folding device (not shown) arranged at the step SB folds the front-side material portion 31aZ inward in the CD direction at a folding position that is a CD-direction central position CL31aZ (corresponding to a boundary position) in the front-side material portion 31aZ, and therefore a CD-direction outward portion 31aZ2 (corresponding to a second portion) of the front-side material portion 31Az becomes overlapped with a CD-direction inward portion 31aZ1 (corresponding to a first portion). Similarly, a known folding device (not shown) arranged at the step SB folds the back-side material portion 41aZ inward in the CD direction at a folding position that is a CD-direction central position CL41aZ (corresponding to a boundary position) in the back-side material portion 41aZ, and therefore a CD-direction outward portion 41aZ2 (corresponding to a second portion) of the back-side material portion 41Az becomes overlapped with a CD-direction inward portion 41aZ1 (corresponding to a first portion). This therefore forms a portion 31ap that is to become the front-band-member continuous body 31a on the one side in the CD direction, and forms a portion 41ap that is to become the back-band-member continuous body 41a on the other side.

Note that when the above-described folding is performed, the elastic-string continuous bodies 35a, 35a... (45a, 45a...) are inserted between the outward portion 31aZ2 (41aZ2) and the inward portion 31aZ1 (41aZ1) in a state of being continuous in the direction of transport, stretched in the direction of transport, and side-by-side in the CD direction, and a hot-melt adhesive has been applied to these elastic-string continuous bodies 35a (45a) or to the base sheet 30mf3 at a position that is upstream of the inserting position in the direction of transport. Accordingly, the elastic-string continuous bodies 35a (45a) are fixed to the base sheet 30mf3 by this adhesive.

According to this manufacturing method of the second embodiment, the one base sheet 30mf3 can be used to manufacture both the front-band-member continuous body 31a and the back-band-member continuous body 41a as members that each have a two-layer structure. Accordingly, there is no need to provide two of the same device in order to give each of the continuous bodies 31a and 41a a two-layer structure. For example, there is no need to provide two feeding devices for feeding nonwoven fabric from original fabric, such as one for the nonwoven fabric for the first layer and another one for the nonwoven fabric for the second layer, that is to say, it is sufficient to provide one feeding device for the base sheet 30mf3. As a result, it is possible to reduce the number of devices, and reduce the equipment cost.

Also, according to this manufacturing method, the inward portion 31aZ1 and the outward portion 31aZ2, which are to become the front-band-member continuous body 31a, can be firmly engaged with each other in the edge portions he of the air holes h. This will be described in detail below. First, when the inward portion 31aZ1 and the outward portion 31aZ2 are overlaid on each other, if characteristics such as the density (g/cm³) of the nonwoven fabric sheets are the same or similar at the surfaces that come into contact with each other, the constituent fibers more easily become entangled in the edge portions he of the air holes h (see the enlarged view of a relevant portion in FIG. 11) when the air holes h are formed. As a result, the inward portion 31aZ1 and the outward portion 31aZ2 become engaged with each other with a high degree of engagement in the edge portions he. There are also cases where characteristics such as the density are different between the two surfaces of the base sheet 30mf3, but in such cases, according to the above configuration, when the inward portion 31aZ1 and the outward portion 31aZ2 are folded at the folding positions, portions of the same one of the two surfaces of the base sheet 30mf3 face each other and come into contact with each other. Accordingly, surfaces of the inward portion 31aZ1 and the outward portion 31aZ2 that have the same characteristics as each other come into contact with each other, thus making it possible for the inward portion 31aZ1 and the outward portion 31aZ2 to become firmly engaged with each other at these surfaces in the edge portions he of the air holes h. Note that the above description similarly applies to the inward portion 41aZ1 and the outward portion 41aZ2 that are to become the back-band-member continuous body 41a.

It should be noted that from the viewpoint of improving this engagement, even if the front-side material portion 31aZ and the back-side material portion 41aZ in FIG. 17 are not integrated as a single piece, it is possible to achieve the effect of improving the engagement. For this reason, if it is sufficient to merely be able to achieve this effect, a configuration is possible in which two base sheets are prepared in advance in a state where the above-described base sheet 30mf3 is cut at a boundary position BL2 between the front-side material portion 31aZ and the back-side material portion 41aZ. In other words, a base sheet for the front-side material portion and a base sheet for the back-side material portion may be separately prepared as members that are separate from each other.

### Third Embodiment

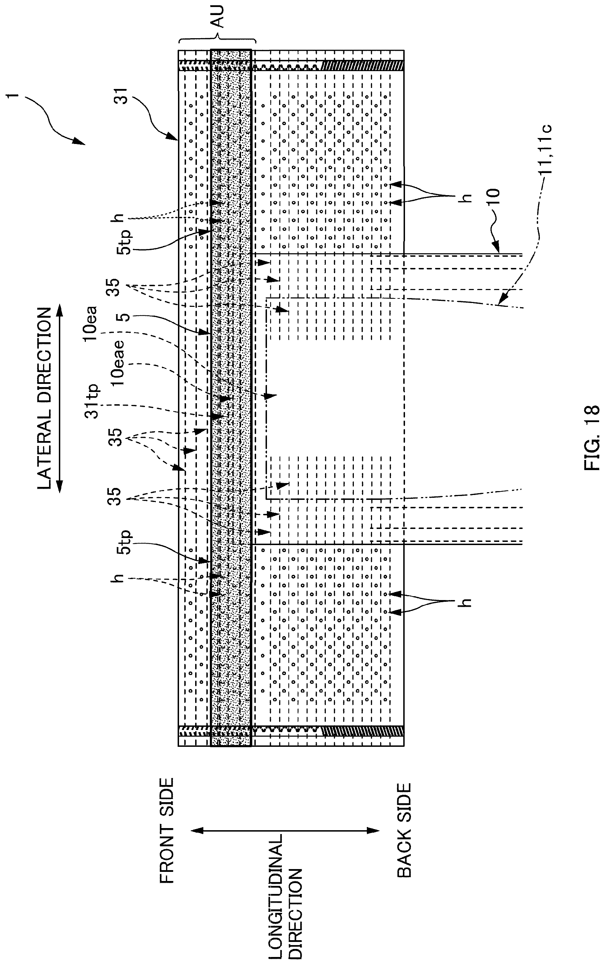

FIG. 18 is a diagram schematically illustrating a manufacturing method of a third embodiment.

In the above-described first embodiment shown in FIG. 3, there is a concern of stressing the wearer's skin on the skin side of the front band member 31 of the diaper 1. Specifically, this front band member 31 has a portion 31tp that projects vertically outward from the vertical end edge portion 10eae of the absorbent main body 10, and here, the end edge portion 10eae of the absorbent main body 10 projects out from this projecting portion 31tp in a step-like manner on the skin side in the thickness direction. For this reason, the wearer can feel stress on their skin at the end edge portion 10eae that projects in a step-like manner.

In view of this, in the third embodiment, a cushioning sheet 5 for reducing skin stress is provided on the skin-side surface of the front band member 31 as shown in the schematic enlarged view from the skin side in FIG. 18. This embodiment is different from the above-described first embodiment mainly with regard to this aspect, and other aspects are substantially the same as in the first embodiment. Accordingly, the same or similar configurations are denoted by the same reference signs, and will not be described further.

As shown in FIG. 18, in the third embodiment as well, the front band member 31 has the portion 31tp that projects vertically outward from the end edge portion 10eae of the absorbent main body 10. However, the cushioning sheet 5 that is made of air-through nonwoven fabric (see the dotted portion in FIG. 18) is arranged on the skin side of the projecting portion 31tp. More specifically, the cushioning sheet 5 is arranged so as to span both the projecting portion 31tp and the end edge portion 10eae of the absorbent main body 10 in the vertical direction, and is fixed to these portions using a hot-melt adhesive or the like. Accordingly, it is possible to reduce the above-described skin stress.

Also, this cushioning sheet 5 has a laterally elongated shape and is longer in the lateral direction than in the vertical direction. For this reason, the sheet 5 projects from the two sides of the absorbent main body 10 in the lateral direction, and in these two laterally projecting portions 5tp, the sheet 5 covers the skin side of several of the air holes h that are formed in the front band member 31. Accordingly, even if elastic strings 35 are located in these air holes h, these elastic strings 35 can be hidden by the cushioning sheet 5 so as to not be visible. Accordingly, the front band member 31 can be given a favorable appearance.

Note that processing for fixing the cushioning sheet 5 is performed between the absorbent main body attaching step S100 and the folding step S110 in FIG. 7. This fixing processing can be realized as described below. Specifically, it can be realized by the cushioning sheet 5 (corresponding to a continuous sheet) that is continuous in the direction of transport being overlaid on and fixed to the skin side of the front-band-member continuous body 31a in FIG. 7 so as to span both sides, in the vertical direction, of the vertical end edge portion 10eae of the absorbent main body 10. A further description for realizing this is thought to be unnecessary. Accordingly, this will not be described further.

Furthermore, although not shown, a similar cushioning sheet 5 may be provided on the portion of the front band member 31 that is to serve as the waist opening BH, that is to say the upper region AU (FIG. 18) of the front band member 31. In this case as well, the sheet 5 is provided so as to project laterally outward from the absorbent main body 10, and in these two laterally projecting portions 5tp, the sheet 5 covers the skin side of several of the air holes h that are formed in the upper region AU of the front band member 31. Accordingly, it is possible to achieve a similar effect of improving the appearance of the front band member 31.

Also, although not shown, in some cases, the sheet 5 that covers several of the air holes h as described above may be overlaid on and fixed to the front band member 31 on the non-skin side of the front band member 31.

Furthermore, needless to say, the sheet 5 may be provided on the back band member 41, and a method for realizing this can be easily arrived at based on the above description, and therefore will not be described here.

### Other embodiments

Although the embodiment of the present disclosure have been described hereinabove, the above embodiment of the present disclosure are simply to facilitate understanding of the present disclosure and are not in any way to be construed as limiting the present disclosure. The present disclosure may variously be changed or altered without departing from its gist and encompass equivalents thereof. For example, deformation which will be described below is possible.

The air holes h are illustrated as examples of hole portions in the above embodiment, and these air holes h are through holes, but there is no limitation whatsoever to this. For example, the hole portions may be bottomed holes that have bottom portions. In the case of bottomed holes, given that they have bottom portions, it is possible to more reliably prevent the problem that the elastic strings 35 (45) can be seen through the holes. It should be noted that the formation of bottomed holes can be realized by passing the above-described sheet-like member 30mf between an emboss roll and an anvil roll (not shown) that are driven to rotate with their outer circumferential surfaces facing each other. Note that the emboss roll is a roll that has multiple protruding portions, which have a substantially flat top face, on the outer circumferential surface, and the anvil roll is a roll that has a substantially smooth outer circumferential surface.

Although the elastic-string continuous bodies 35a and 45a are illustrated as examples of the continuous elastic members in the above embodiment, there is no limitation whatsoever to this. For example, they may be elastic belt continuous bodies.

Each of the pin members 55p of the intermediate roller 55 has the conical portion 55pa and the circular column portion 55pb as shown in FIG. 11 in the above embodiment, but there is no limitation whatsoever to this. For example, instead of the conical portion 55pa, it may have a pyramid-shaped portion that has a polygonal cross-section, such as a triangular pyramid or a quadrangular pyramid, and instead of the circular column portion 55pb, it may have a columnar portion that has a polygonal cross-section, such as a triangular column or a quadrangular column.

In the above embodiment, the sheet-like member 30mf serving as the intermediate product 1m is wrapped around both the upstream roller 51 and the intermediate roller 55 of the air-hole forming device 50 in the air-hole forming step S50 as shown in FIG. 10, but there is no limitation whatsoever to this. Specifically, the sheet-like member 30mf may pass between the rollers 51 and 55 without being wrapped around the upstream roller 51 and the intermediate roller 55.

Although the three-piece type of disposable diaper 1 is illustrated as an example of the absorbent article in the above embodiment, there is no limitation whatsoever to this. For example, the method and device for manufacturing a sheet-like member of the present invention may be applied when manufacturing a sheet-like member that is a material for constituting a two-piece type of disposable diaper. It should be noted that a two-piece type of disposable diaper is a type of diaper that has an exterior sheet as a first component having a front portion, a crotch portion, and a back portion, and the absorbent main body 10 as second component that is fixed to the skin-side surface of the exterior sheet. Also, in this case, the continuous sheet of exterior sheets is formed by two overlapped nonwoven fabric sheets, and the method and device for manufacturing a sheet-like member of the present invention is used when forming hole portions in the two overlapped nonwoven fabric sheets and cutting elastic members arranged between the two overlapped nonwoven fabric sheets.

Furthermore, this two-piece type of diaper may be a so-called tape-type disposable diaper. Note that a tape-type disposable diaper is a type of diaper that uses fastening tape in order to connect the front portion, which covers the front side of the trunk portion of the wearer, to the back portion, which cover the back side of the trunk portion.

Furthermore, the absorbent article is not limited whatsoever to being the disposable diaper 1. Specifically, the method and device for manufacturing a sheet-like member of the present invention is applied when manufacturing any absorbent article that is manufactured with use of nonwoven fabric sheets. For this reason, the concept of this absorbent article also encompasses a urine absorbing pad, a sanitary napkin, and the like.

In the above embodiment, as shown in FIG. 9B, the elastic-string continuous bodies 35a, which are one example of continuous elastic members, are cut at the one position PC in the direction of transport in the non-fixed region AL1 that corresponds to the non-stretchy region AL, and the cut elastic strings 35 and elastic-string continuous bodies 35a contract toward the fixed region AH1 that corresponds to the stretchy region AH, thus forming the non-stretchy region AL, but there is no limitation whatsoever to this.

For example, in a region AL1 that corresponds to the non-stretchy region AL in FIG. 9A, the elastic-string continuous bodies 35a may be cut at multiple positions PC, PC... in the direction of transport such that the elastic-string continuous bodies 35a are cut into small pieces in the region AL1, thereby forming the non-stretchy region AL from the region AL1. Note that in this case, before being cut, the elastic-string continuous bodies 35a may be fixed with a hot-melt adhesive not only in regions AH1 that correspond to the stretchy regions AH, but also in the region AL1 that corresponds to the non-stretchy region AL.

Although the position of the air-hole forming step S50 is set upstream of the position of the slitting step S60 in the direction of transport in the above embodiment, there is no limitation whatsoever to this. For example, the air-hole forming step S50 may be set at a position that is downstream of the position of the slitting step S60 in the direction of transport.

Although the positions of the front-side elastic string cutting step S80 and the back-side elastic string cutting step S90 are set downstream of the position of the slitting step S60 in the direction of transport in the above embodiment, there is no limitation whatsoever to this. For example, the positions of the front-side elastic string cutting step S80 and the back-side elastic string cutting step S90 may be set upstream of the position of the slitting step S60 in the direction of transport.

### [Reference Signs List]

1 disposable diaper (absorbent article),
1a diaper continuous body, 1Ha approximately ladder-shaped diaper continuous body,
1m intermediate product,
5 cushioning sheet, 5tp portion,
10 absorbent main body,
10LG portion, 10ea end portion, 10eae end edge portion,
10eb end portion,
11 absorbent body, 11c absorbent core,
13 top sheet, 15 back sheet,
15a leak-proof sheet, 15b exterior sheet,
16 elastic string,
30mf sheet-like member (composite sheet),
30mf1 continuous nonwoven fabric sheet (first nonwoven fabric sheet), 30mf2 continuous nonwoven fabric sheet (second nonwoven fabric sheet), 30mf2p1 portion, 30mf2p2 portion,
30mf3 base sheet,
31 front band member, 31a front-band-member continuous body,
31aZ front-side material portion (third portion),
31aZ1 inward portion (first portion), 31aZ2 outward portion (second portion),
31ap portion, 31e end portion,
31s no-absorbent-body portion, 31tp portion,
32 nonwoven fabric sheet, 32Le end portion,
33 nonwoven fabric sheet, 33B folded portion,
35 elastic string (elastic member), 35eu portion,
35a elastic-string continuous body (elastic member),
35aed end portion, 35aedn portion,
41 back band member, 41a back-band-member continuous body,
41aZ back-side material portion (third portion),
41aZ1 inward portion (first portion), 41aZ2 outward portion (second portion),
41ap portion,
42 nonwoven fabric sheet, 42Le end portion,
43 nonwoven fabric sheet, 43B folded portion,
45 elastic string (elastic member),
45a elastic-string continuous body (elastic member),
50 air hole forming device (hole-portion forming device), 51 upstream roll, 51h hole portion,
55 intermediate roll,
55p pin member (press-in member), 55pa conical portion, 55pb circular column portion,
55r receiving portion, 55rt top face,
58 downstream roll, 58h hole portion,
60 slitter device,
80 front-side cutter device (elastic-member cutting device),
81u cutter roll, 81ua outer circumferential surface,
81d anvil roll, 81da outer circumferential surface,
82 bearing member,
84 cutter block, 85 housing,
90 back-side cutter device (elastic-member cutting device)
B burr, C cutter blade, h air hole (hole portion), he edge portion,
AH stretchy region (high-stretch region), AH1 fixed region (region corresponding to high-stretch region),
AL non-stretchy region (low-stretch region) , AL1 non-fixed region (region corresponding to low-stretch region),
AU upper region, AD lower region, ADc central region, ADe end region, BH waist opening, LH leg opening, LG leg gather,
CL1 central position, CL10 predetermined position,
SS side seal portion, PC predetermined position, TC mark,
Gh31 air hole group, Rh31 air hole row,
Gh41 air hole group, Rh41 air hole row,
G51h1 hole portion group, G51h2 hole portion group,
G55p1 pin member group, G55p2 pin member group,
G58h1 hole portion group, G58h2 hole portion group,
SG51h1 hole portion group set, SG51h2 hole portion group set,
SG55p1 pin member group set, SG55p2 pin member group set,
R51 first transporting route, R55 second transporting route, R58 third transporting route,
P51 closest position (hole-portion formation position), P55 closest position,
P81 closest position (cutting position),
S50 air-hole forming step (hole-portion forming step), S60 slitting step, S70 gap forming step,
S80 front-side elastic string cutting step (elastic-member cutting step), S90 back-side elastic string cutting step (elastic-member cutting step), S100 absorbent main body attaching step (absorbent-main-body fixing step),
S110 folding step, S120 side sealing step, S130 cutting step,
SB folding step,
CL31aZ central position (folding position, boundary position),
CL41aZ central position (folding position, boundary position),
BL boundary position, BL2 boundary position

## Claims

1. A method for manufacturing a sheet-like member (30mf) for an absorbent article (1) from a composite sheet,
the sheet-like member (30mf) having a high-stretch region (AH) and a low-stretch region (AL) side-by-side in a predetermined direction,
the low-stretch region (AL) having a lower stretchability in the predetermined direction than the high-stretch region (AH),
a plurality of hole portions (h) being formed in at least the high-stretch region (AH),
the method comprising:
a transporting step of transporting the composite sheet in a direction of transport that is the predetermined direction,
the composite sheet being constituted by a first nonwoven fabric sheet (30mf1), a second nonwoven fabric sheet (30mf2), and a plurality of elastic members (35a),
the first nonwoven fabric sheet (30mf1) being continuous in the predetermined direction,
the second nonwoven fabric sheet (30mf2) being continuous in the predetermined direction and arranged overlapped with the first nonwoven fabric sheet (30mf1) in a thickness direction of the composite sheet,
the plurality of elastic members (35a) being stretched in the predetermined direction and extending along the predetermined direction,
the plurality of elastic members (35a) being inserted between the first nonwoven fabric sheet (30mf1) and the second nonwoven fabric sheet (30mf2) in a side-by-side arrangement in a CD direction and being fixed in at least a region (AH1) corresponding to the high-stretch region (AH),
the CD direction being a direction that intersects the predetermined direction;
a hole-portion forming step (S50) of forming the hole portions (h) in the composite sheet in the region (AH1) corresponding to the high-stretch region (AH), and engaging the first nonwoven fabric sheet (30mf1) and the second nonwoven fabric sheet (30mf2) with each other in at least edge portions (he) of the hole portions (h),
the hole portions (h) extending in the thickness direction,
the forming of the hole portions (h) being performed by pressing a press-in member (55p) in the thickness direction at positions between a pair of elastic members (35a) that are adjacent in the CD direction and that are in at least the region (AH1) corresponding to the high-stretch region (AH); and
an elastic-member cutting step (S80, S90) of cutting the plurality of elastic members (35a) in a region (AL1) corresponding to the low-stretch region (AL), forming the high-stretch region (AH) and the low-stretch region (AL) side-by-side in the direction of transport in the composite sheet,
the cutting being performed at a position that is downstream of a hole-portion formation position (P51) in the direction of transport,
the elastic members (35a) being prevented from being visible through the hole portions (h), and
the hole-portion formation position (P51) being a position at which formation of the hole portions (h) in the hole-portion forming step (S50) is performed.

2. The method for manufacturing a sheet-like member for an absorbent article according to claim 1, wherein
the composite sheet has a base sheet (30mf3) that is continuous in the direction of transport,
the base sheet has a first portion (31aZ1, 41aZ1) that is to become the first nonwoven fabric sheet (30mf1) and a second portion (31aZ2, 41aZ2) that is to become the second nonwoven fabric sheet (30mf2), the first portion and the second portion being integrated as a single piece and adjacent in the CD direction, and
the method further comprises a folding step (SB) of, at a position that is upstream in the direction of transport of the hole-portion formation position (P51) of the hole-portion forming step (S50), folding the base sheet (30mf3) in the CD direction at a folding position that is a boundary position between the first portion and the second portion such that the first nonwoven fabric sheet (30mf1) and the second nonwoven fabric sheet (30mf2) become overlapped.

3. The method for manufacturing a sheet-like member for an absorbent article according to claim 2, wherein
letting a third portion (31aZ, 41aZ) be a portion in which the first portion (31aZ1, 41aZ1) and the second portion (32aZ1, 42aZ2) are integrated as a single piece,
the base sheet (30mf3) has the third portion (31aZ, 41aZ) on each of two sides in the CD direction, and
in the folding step (SB), the base sheet (30mf3) is folded at the folding position that is located on each of the two sides in the CD direction.

4. The method for manufacturing a sheet-like member for an absorbent article according to any of claims 1 to 3, wherein
the region (AH1) corresponding to the high-stretch region (AH) has a fixing portion for fixing the elastic members (35a),
the first nonwoven fabric sheet (30mf1) and the second nonwoven fabric sheet (30mf2) are fixed to each other in the fixing portion, and
besides the fixing portion and the edge portions (he) of the hole portions (h), the region (AH1) corresponding to the high-stretch region (AH) does not have a fixing portion that fixes the first nonwoven fabric sheet (30mf1) and the second nonwoven fabric sheet (30mf2) to each other.

5. The method for manufacturing a sheet-like member for an absorbent article according to claim 4, wherein
the fixing portion is an adhesive applied to the elastic members (35a), and
the adhesive is not provided in the edge portions (he).

6. The method for manufacturing a sheet-like member for an absorbent article according to any of claims 1 to 5, wherein
the method further comprises an absorbent-main-body fixing step (S100) of fixing the absorbent main body (10) for the absorbent article (1) to the low-stretch region (AL) of the composite sheet, at a position that is downstream in the direction of transport of a cutting position (P81),
the cutting position (P81) being a position at which the elastic members (35a) are cut in the elastic-member cutting step (S80, S90).

7. The method for manufacturing a sheet-like member for an absorbent article according to any of claims 1 to 6, wherein
the method further comprises a continuous-sheet fixing step of fixing a continuous sheet (5) that is continuous in the predetermined direction to the composite sheet, at a position that is downstream in the direction of transport of the hole-portion formation position (P51) of the hole-portion forming step (S50), and
in the continuous-sheet fixing step,
the continuous sheet (5) is overlaid in the thickness direction so as to cover several of the hole portions (h).

8. The method for manufacturing a sheet-like member for an absorbent article according to any of claims 1 to 7, wherein
the elastic members (35a) are cut by cutter blades (C) being pressed into the composite sheet, and a cutting mark (TC) is formed in the composite sheet, and
the cutting is performed such that the mark (TC) is not overlapped in the CD direction with, among the plurality of hole portions (h), a pair of hole portions (h) that are located closest to the mark (TC) on two sides in the direction of transport.

9. The method for manufacturing a sheet-like member for an absorbent article according to claim 8, wherein
a size (LTC) of the mark (TC) in the direction of transport is smaller than a distance (Dh) in the direction of transport between, among the plurality of hole portions (h), the pair of hole portions (h) that are located closest to the mark (TC) on the two sides in the direction of transport.

10. The method for manufacturing a sheet-like member for an absorbent article according to any of claims 1 to 9, wherein
the hole portions (h) are through holes that pass through the composite sheet in the thickness direction.

11. The method for manufacturing a sheet-like member for an absorbent article according to any of claims 1 to 9, wherein
the hole portions (h) are bottomed holes that each have a bottom portion.

12. A device for manufacturing a sheet-like member (30mf) for an absorbent article (1) from a composite sheet,
the sheet-like member (30mf) having a high-stretch region (AH) and a low-stretch region (AL) side-by-side in a predetermined direction,
the low-stretch region (AL) having a lower stretchability in the predetermined direction than the high-stretch region (AH),
a plurality of hole portions (h) being formed in at least the high-stretch region (AH),
the device comprising:
a transporting device for transporting the composite sheet in a direction of transport that is the predetermined direction,
the composite sheet being constituted by a first nonwoven fabric sheet (30mf1), a second nonwoven fabric sheet (30mf2), and a plurality of elastic members (35a),
the first nonwoven fabric sheet (30mf1) being continuous in the predetermined direction,
the second nonwoven fabric sheet (30mf2) being continuous in the predetermined direction and arranged overlapped with the first nonwoven fabric sheet (30mf1) in a thickness direction of the composite sheet,
the plurality of elastic members (35a) being stretched in the predetermined direction and extending along the predetermined direction,
the plurality of elastic members (35a) being inserted between the first nonwoven fabric sheet (30mf1) and the second nonwoven fabric sheet (30mf2) in a side-by-side arrangement in a CD direction and being fixed in at least a region (AH1) corresponding to the high-stretch region (AH),
the CD direction being a direction that intersects the predetermined direction;
a hole-portion forming device (50) for forming the hole portions (h) in the composite sheet in the region corresponding to the high-stretch region (AH), and engaging the first nonwoven fabric sheet (30mf1) and the second nonwoven fabric sheet (30mf2) with each other in at least edge portions (he) of the hole portions (h),
the hole portions (h) extending in the thickness direction,
the forming of the hole portions (h) being performed by pressing a press-in member (55p) in the thickness direction at positions between a pair of elastic members (35a) that are adjacent in the CD direction and that are in at least the region (AH1) corresponding to the high-stretch region (AH); and
an elastic-member cutting device (80, 90) for cutting the plurality of elastic members (35a) in a region (AL1) corresponding to the low-stretch region (AL), forming the high-stretch region (AH) and the low-stretch region (AL) side-by-side in the direction of transport in the composite sheet,
the cutting being performed at a position that is downstream of a hole-portion formation position (P51) in the direction of transport,
the elastic members (35a) being prevented from being visible through the hole portions (h), and
the hole-portion formation position (P51) being a position at which formation of the hole portions (h) by the hole-portion forming device (50) is performed.

## Patentansprüche

1. Verfahren für die Herstellung eines lagenartigen Elements (30mf) für einen absorbierenden Artikel (1) aus einer Verbundlage,
wobei das lagenartige Element (30mf) einen Bereich hoher Dehnung (AH) und einen Bereich geringer Dehnung (AL) nebeneinander in einer vorgegebenen Richtung aufweist,
der Bereich geringer Dehnung (AL) in der vorgegebenen Richtung eine geringere Dehnbarkeit aufweist als der Bereich hoher Dehnung (AH),
eine Vielzahl von Lochteilen (h) in zumindest dem Bereich hoher Dehnung (AH) ausgebildet ist,
wobei das Verfahren Folgendes umfasst:
einen Transportschritt des Transportierens der Verbundlage in einer Transportrichtung, die die vorgegebene Richtung ist,
wobei die Verbundlage aus einer ersten Vliesstofflage (30mf1), einer zweiten Vliesstofflage (30mf2) und einer Vielzahl von elastischen Elementen (35a) besteht,
wobei die erste Vliesstofflage (30mf1) in der vorgegebenen Richtung fortlaufend ist,
die zweite Vliesstofflage (30mf2) in der vorgegebenen Richtung fortlaufend ist und mit der ersten Vliesstofflage (30mf1) überlappend in einer Dickenrichtung der Verbundlage angeordnet ist,
die Vielzahl von elastischen Elementen (35a) in der vorgegebenen Richtung gedehnt ist und sich entlang der vorgegebenen Richtung erstreckt,
die Vielzahl von elastischen Elementen (35a) zwischen der ersten Vliesstofflage (30mf1) und der zweiten Vliesstofflage (30mf2) in einer Anordnung nebeneinander in einer Querrichtung (CD-Richtung) eingefügt ist und in zumindest einem Bereich (AH1), der dem Bereich hoher Dehnung (AH) entspricht, befestigt ist,
die CD-Richtung eine Richtung ist, die die vorgegebene Richtung kreuzt;
einen Lochteilbildungsschritt (S50) zur Bildung der Lochteile (h) in der Verbundlage in dem Bereich (AH1), der dem Bereich hoher Dehnung (AH) entspricht, und zum Eingreifen der ersten Vliesstofflage (30mf1) und der zweiten Vliesstofflage (30mf2) ineinander in zumindest Randteilen (he) der Lochteile (h),
wobei sich die Lochteile (h) in der Dickenrichtung erstrecken,
die Bildung der Lochteile (h) durch Pressen eines Einpresselements (55p) in der Dickenrichtung an Positionen zwischen einem Paar von elastischen Elementen (35a) durchgeführt wird ,die in der CD-Richtung benachbart sind und die in zumindest dem Bereich (AH1) vorliegen, der dem Bereich hoher Dehnung (AH) entspricht; und
einen Schritt des Schneidens elastischer Elemente (S80, S90) des Schneidens der Vielzahl von elastischen Elementen (35a) in einem Bereich (AL1), der dem Bereich geringer Dehnung (AL) entspricht, unter Bildung des Bereichs hoher Dehnung (AH) und des Bereichs geringer Dehnung (AL) nebeneinander in der Transportrichtung in der Verbundlage,
wobei das Schneiden an einer Position durchgeführt wird, die nachgelagert von einer Lochteilbildungsposition (P51) in der Transportrichtung vorliegt,
verhindert wird, dass die elastischen Elemente (35a) durch die Lochteile (h) sichtbar sind, und
die Lochteilbildungsposition (P51) eine Position ist, an der die Bildung der Lochteile (h) in dem Lochteilbildungsschritt (S50) durchgeführt wird.

2. Verfahren für die Herstellung eines lagenartigen Elements für einen absorbierenden Artikel nach Anspruch 1, wobei
die Verbundlage eine Grundlage (30mf3) aufweist, die in der Transportrichtung fortlaufend ist,
die Grundlage Folgendes aufweist: ein erstes Teil (31aZ1, 41aZ1), das die erste Vliesstofflage (30mf1) wird, und ein zweites Teil (31aZ2, 41aZ2), das die zweite Vliesstofflage (30mf2) wird, wobei das erste Teil und das zweite Teil zu einem Einzelstück eingebunden werden und benachbart in der CD-Richtung vorliegen, und
das Verfahren weiter Folgendes umfasst: einen Faltschritt (SB), an einer Position, die in der Transportrichtung von der Lochteilbildungsposition (P51) des Lochteilbildungsschritts (S50) vorgelagert ist, des Faltens der Grundlage (30mf3) in der CD-Richtung an einer Faltposition, die eine Grenzposition zwischen dem ersten Teil und dem zweiten Teil ist, sodass die erste Vliesstofflage (30mf1) und die zweite Vliesstofflage (30mf2) überlappt werden.

3. Verfahren für die Herstellung eines lagenartigen Elements für einen absorbierenden Artikel nach Anspruch 2, wobei
ein drittes Teil (31aZ, 41aZ) ein Teil sein wird, in dem das erste Teil (31aZ1, 41aZ1) und das zweite Teil (32aZ1, 42aZ2) zu einem Einzelstück eingebunden sind,
wobei die Grundlage (30mf3) das dritte Teil (31aZ, 41aZ) auf jeder der zwei Seiten in der CD-Richtung aufweist, und
in dem Faltschritt (SB) die Grundlage (30mf3) an der Faltposition gefaltet wird, die sich auf jeder der zwei Seiten in der CD-Richtung befindet.

4. Verfahren für die Herstellung eines lagenartigen Elements für einen absorbierenden Artikel nach einem der Ansprüche 1 bis 3, wobei
der Bereich (AH1), der dem Bereich hoher Dehnung (AH) entspricht, ein Befestigungsteil für die Befestigung der elastischen Elemente (35a) aufweist,
die erste Vliesstofflage (30mf1) und die zweite Vliesstofflage (30mf2) in dem Befestigungsteil aneinander befestigt sind und
der Bereich (AH1), der dem Bereich hoher Dehnung (AH) entspricht, außer dem Befestigungsteil und den Randteilen (he) der Lochteile (h) kein Befestigungsteil aufweist, das die erste Vliesstofflage (30mf1) und die zweite Vliesstofflage (30mf2) aneinander befestigt.

5. Verfahren für die Herstellung eines lagenartigen Elements für einen absorbierenden Artikel nach Anspruch 4, wobei
das Befestigungsteil ein Haftmittel ist, das auf die elastischen Elemente (35a) aufgetragen ist, und
das Haftmittel nicht in den Randteilen (he) bereitgestellt ist.

6. Verfahren für die Herstellung eines lagenartigen Elements für einen absorbierenden Artikel nach einem der Ansprüche 1 bis 5, wobei
das Verfahren weiter einen Befestigungsschritt eines absorbierenden Hauptkörpers (S100) des Befestigens des absorbierenden Hauptkörpers (10) für den absorbierenden Artikel (1) an dem Bereich geringer Dehnung (AL) der Verbundlage an einer Position umfasst, die in der Transportrichtung von einer Schnittposition (P81) nachgelagert ist,
wobei die Schnittposition (P81) eine Position ist, an der die elastischen Elemente (35a) in dem Schritt des Schneidens elastischer Elemente (S80, S90) geschnitten werden.

7. Verfahren für die Herstellung eines lagenartigen Elements für einen absorbierenden Artikel nach einem der Ansprüche 1 bis 6, wobei
das Verfahren weiter einen Befestigungsschritt einer fortlaufenden Lage des Befestigens einer fortlaufenden Lage (5), die in der vorgegebenen Richtung fortlaufend ist, an der Verbundlage an einer Position umfasst, die in der Transportrichtung von der Lochteilbildungsposition (P51) des Lochteilbildungsschritts (S50) nachgelagert ist, und
in dem Befestigungsschritt einer fortlaufenden Lage
die fortlaufende Lage (5) in der Dickenrichtung überlagert ist, sodass mehrere der Lochteile (h) bedeckt werden.

8. Verfahren für die Herstellung eines lagenartigen Elements für einen absorbierenden Artikel nach einem der Ansprüche 1 bis 7, wobei
die elastischen Elemente (35a) durch Schneidmesser (C), die in die Verbundlage gedrückt werden, geschnitten werden und eine Schnittmarkierung (TC) in der Verbundlage ausgebildet wird und
das Schneiden derart durchgeführt wird, dass die Markierung (TC) in der CD-Richtung nicht, unter der Vielzahl von Lochteilen (h), mit einem Paar von Lochteilen (h) überlappt, die sich am nächsten zu der Markierung (TC) auf zwei Seiten in der Transportrichtung befinden.

9. Verfahren für die Herstellung eines lagenartigen Elements für einen absorbierenden Artikel nach Anspruch 8, wobei
eine Größe (LTC) der Markierung (TC) in der Transportrichtung kleiner ist als ein Abstand (Dh) in der Transportrichtung zwischen, unter der Vielzahl von Lochteilen (h), dem Paar von Lochteilen (h), die sich am nächsten zu der Markierung (TC) auf den zwei Seiten in der Transportrichtung befinden.

10. Verfahren für die Herstellung eines lagenartigen Elements für einen absorbierenden Artikel nach einem der Ansprüche 1 bis 9, wobei
die Lochteile (h) Durchgangslöcher sind, die durch die Verbundlage in der Dickenrichtung verlaufen.

11. Verfahren für die Herstellung eines lagenartigen Elements für einen absorbierenden Artikel nach einem der Ansprüche 1 bis 9, wobei
die Lochteile (h) Löcher mit Boden sind, die jeweils ein Bodenteil aufweisen.

12. Vorrichtung für die Herstellung eines lagenartigen Elements (30mf) für einen absorbierenden Artikel (1) aus einer Verbundlage,
wobei das lagenartige Element (30mf) einen Bereich hoher Dehnung (AH) und einen Bereich geringer Dehnung (AL) nebeneinander in einer vorgegebenen Richtung aufweist,
der Bereich geringer Dehnung (AL) in der vorgegebenen Richtung eine geringere Dehnbarkeit aufweist als der Bereich hoher Dehnung (AH),
eine Vielzahl von Lochteilen (h) in zumindest dem Bereich hoher Dehnung (AH) ausgebildet ist,
wobei die Vorrichtung Folgendes umfasst:
eine Transportvorrichtung für das Transportieren der Verbundlage in einer Transportrichtung, die die vorgegebene Richtung ist,
wobei die Verbundlage aus einer ersten Vliesstofflage (30mf1), einer zweiten Vliesstofflage (30mf2) und einer Vielzahl von elastischen Elementen (35a) besteht,
wobei die erste Vliesstofflage (30mf1) in der vorgegebenen Richtung fortlaufend ist,
die zweite Vliesstofflage (30mf2) in der vorgegebenen Richtung fortlaufend ist und mit der ersten Vliesstofflage (30mf1) überlappend in einer Dickenrichtung der Verbundlage angeordnet ist,
die Vielzahl von elastischen Elementen (35a) in der vorgegebenen Richtung gedehnt ist und sich entlang der vorgegebenen Richtung erstreckt,
die Vielzahl von elastischen Elementen (35a) zwischen der ersten Vliesstofflage (30mf1) und der zweiten Vliesstofflage (30mf2) in einer Anordnung nebeneinander in einer CD-Richtung eingefügt ist und in zumindest einem Bereich (AH1), der dem Bereich hoher Dehnung (AH) entspricht, befestigt ist,
die CD-Richtung eine Richtung ist, die die vorgegebene Richtung kreuzt;
eine Lochteilbildungsvorrichtung (50) für die Bildung der Lochteile (h) in der Verbundlage in dem Bereich, der dem Bereich hoher Dehnung (AH) entspricht, und für das Eingreifen der ersten Vliesstofflage (30mf1) und der zweiten Vliesstofflage (30mf2) ineinander in zumindest Randteilen (he) der Lochteile (h),
wobei sich die Lochteile (h) in der Dickenrichtung erstrecken,
die Bildung der Lochteile (h) durch Pressen eines Einpresselements (55p) in der Dickenrichtung an Positionen zwischen einem Paar von elastischen Elementen (35a) durchgeführt wird, die in der CD-Richtung benachbart sind und die in zumindest dem Bereich (AH1) vorliegen, der dem Bereich hoher Dehnung (AH) entspricht; und
eine Schneidvorrichtung für elastische Elemente (80, 90) für das Schneiden der Vielzahl von elastischen Elementen (35a) in einem Bereich (AL1), der dem Bereich geringer Dehnung (AL) entspricht, unter Bildung des Bereichs hoher Dehnung (AH) und des Bereichs geringer Dehnung (AL) nebeneinander in der Transportrichtung in der Verbundlage,
wobei das Schneiden an einer Position durchgeführt wird, die nachgelagert von einer Lochteilbildungsposition (P51) in der Transportrichtung vorliegt,
verhindert wird, dass die elastischen Elemente (35a) durch die Lochteile (h) sichtbar sind, und
die Lochteilbildungsposition (P51) eine Position ist, an der die Bildung der Lochteile (h) durch Lochteilbildungsvorrichtung (50) durchgeführt wird.

## Revendications

1. Procédé de fabrication d'un élément de type feuille (30mf) pour un article absorbant (1) à partir d'une feuille composite,
l'élément de type feuille (30mf) ayant une région d'extensibilité élevée (AH) et une région de faible extensibilité (AL) côte à côte dans une direction prédéterminée,
la région de faible extensibilité (AL) ayant une extensibilité plus faible dans la direction prédéterminée par rapport à la région d'extensibilité élevée (AH),
une pluralité de parties formant trou (h) étant formées dans au moins la région d'extensibilité élevée (AH),
le procédé comportant :
une étape de transport consistant à transporter la feuille composite dans une direction de transport qui est la direction prédéterminée,
la feuille composite étant constituée par une première feuille de tissu non tissé (30mf1), une deuxième feuille de tissu non tissé (30mf2), et une pluralité d'éléments élastiques (35a),
la première feuille de tissu non tissé (30mf1) étant continue dans la direction prédéterminée,
la deuxième feuille de tissu non tissé (30mf2) étant continue dans la direction prédéterminée et étant agencée de manière à chevaucher la première feuille de tissu non tissé (30mf1) dans une direction allant dans le sens de l'épaisseur de la feuille composite,
les éléments élastiques de la pluralité d'éléments élastiques (35a) étant étirés dans la direction prédéterminée et s'étendant le long de la direction prédéterminée,
les éléments élastiques de la pluralité d'éléments élastiques (35a) étant insérés entre la première feuille de tissu non tissé (30mf1) et la deuxième feuille de tissu non tissé (30mf2) selon un agencement côte à côte dans une direction allant dans le sens travers et étant fixés dans au moins une région (AH1) correspondant à la région d'extensibilité élevée (AH),
la direction allant dans le sens travers étant une direction qui croise la direction prédéterminée ;
une étape de formation de parties formant trou (S50) consistant à former des parties formant trou (h) dans la feuille composite dans la région (AH1) correspondant à la région d'extensibilité élevée (AH), et à mettre en prise la première feuille de tissu non tissé (30mf1) et la deuxième feuille de tissu non tissé (30mf2) l'une par rapport à l'autre dans au moins des parties formant bord (he) des parties formant trou (h),
les parties formant trou (h) s'étendant dans la direction allant dans le sens de l'épaisseur,
la formation des parties formant trou (h) étant effectuée en enfonçant un élément d'enfoncement (55p) dans la direction allant dans le sens de l'épaisseur au niveau de positions entre une paire d'éléments élastiques (35a) qui sont adjacents dans la direction allant dans le sens travers et qui sont dans au moins la région (AH1) correspondant à la région d'extensibilité élevée (AH) ; et
une étape de découpe d'éléments élastiques (S80, S90) consistant à découper la pluralité d'éléments élastiques (35a) dans une région (AL1) correspondant à la région de faible extensibilité (AL), pour former la région d'extensibilité élevée (AH) et la région de faible extensibilité (AL) côte à côte dans la direction de transport dans la feuille composite,
la découpe étant effectuée au niveau d'une position qui est en aval par rapport à une position de formation de parties formant trou (P51) dans la direction de transport,
les éléments élastiques (35a) étant empêchés d'être visibles au travers des parties formant trou (h), et
la position de formation de parties formant trou (P51) étant une position au niveau de laquelle la formation des parties formant trou (h) au cours de l'étape de formation de parties formant trou (S50) est effectuée.

2. Procédé de fabrication d'un élément de type feuille pour un article absorbant selon la revendication 1, dans lequel
la feuille composite a une feuille de base (30mf3) qui est continue dans la direction de transport,
la feuille de base a une première partie (31aZ1, 41aZ1) qui est destinée à devenir la première feuille de tissu non tissé (30mf1) et une deuxième partie (31aZ2, 41aZ2) qui est destinée à devenir la deuxième feuille de tissu non tissé (30mf2), la première partie et la deuxième partie étant intégrées sous la forme d'une seule pièce et adjacentes dans la direction allant dans le sens travers, et
le procédé comporte par ailleurs une étape de pliage (SB) consistant à, au niveau d'une position qui est en amont dans la direction de transport par rapport à la position de formation de parties formant trou (P51) de l'étape de formation de parties formant trou (S50), plier la feuille de base (30mf3) dans la direction allant dans le sens travers au niveau d'une position de pliage qui est une position limite entre la première partie et la deuxième partie de telle sorte que la première feuille de tissu non tissé (30mf1) et la deuxième feuille de tissu non tissé (30mf2) se chevauchent.

3. Procédé de fabrication d'un élément de type feuille pour un article absorbant selon la revendication 2, dans lequel
en laissant une troisième partie (31aZ, 41aZ) être une partie dans laquelle la première partie (31aZ1, 41aZ1) et la deuxième partie (32aZ1, 42aZ2) sont intégrées sous la forme d'une seule pièce,
la feuille de base (30mf3) a la troisième partie (31aZ, 41aZ) sur chacun des deux côtés dans la direction allant dans le sens travers, et
au cours de l'étape de pliage (SB), la feuille de base (30mf3) est pliée au niveau de la position de pliage qui est située sur chacun des deux côtés dans la direction allant dans le sens travers.

4. Procédé de fabrication d'un élément de type feuille pour un article absorbant selon l'une quelconque des revendications 1 à 3, dans lequel
la région (AH1) correspondant à la région d'extensibilité élevée (AH) a une partie de fixation servant à des fins de fixation des éléments élastiques (35a),
la première feuille de tissu non tissé (30mf1) et la deuxième feuille de tissu non tissé (30mf2) sont fixées l'une par rapport à l'autre dans la partie de fixation, et
en plus de la partie de fixation et des parties formant bord (he) des parties formant trou (h), la région (AH1) correspondant à la région d'extensibilité élevée (AH) n'a pas de partie de fixation qui fixe la première feuille de tissu non tissé (30mf1) et la deuxième feuille de tissu non tissé (30mf2) l'une par rapport à l'autre.

5. Procédé de fabrication d'un élément de type feuille pour un article absorbant selon la revendication 4, dans lequel
la partie de fixation est un adhésif appliqué sur les éléments élastiques (35a), et
l'adhésif n'est pas fourni dans les parties formant bord (he).

6. Procédé de fabrication d'un élément de type feuille pour un article absorbant selon l'une quelconque des revendications 1 à 5, dans lequel
le procédé comporte par ailleurs une étape de fixation de corps principal absorbant (S100) consistant à fixer le corps principal absorbant (10) pour l'article absorbant (1) sur la région de faible extensibilité (AL) de la feuille composite, au niveau d'une position qui est en aval dans la direction de transport par rapport à une position de découpe (P81),
la position de découpe (P81) étant une position au niveau de laquelle les éléments élastiques (35a) sont découpés au cours de l'étape de découpe d'éléments élastiques (S80. S90).

7. Procédé de fabrication d'un élément de type feuille pour un article absorbant selon l'une quelconque des revendications 1 à 6, dans lequel
le procédé comporte par ailleurs une étape de fixation de feuille continue consistant à fixer une feuille continue (5) qui est continue dans la direction prédéterminée sur la feuille composite, au niveau d'une position qui est en aval dans la direction de transport par rapport à la position de formation de parties formant trou (P51) de l'étape de formation de parties formant trou (S50), et
au cours de l'étape de fixation de feuille continue,
la feuille continue (5) est superposée dans la direction allant dans le sens de l'épaisseur de manière à recouvrir plusieurs des parties formant trou (h).

8. Procédé de fabrication d'un élément de type feuille pour un article absorbant selon l'une quelconque des revendications 1 à 7, dans lequel
les éléments élastiques (35a) sont découpés par des lames d'outil à couper (C) qui sont enfoncées dans la feuille composite, et une marque de découpe (TC) est formée dans la feuille composite, et
la découpe est effectuée de telle sorte que la marque (TC) n'est pas chevauchée dans la direction allant dans le sens travers par, parmi la pluralité de parties formant trou (h), une paire de parties formant trou (h) qui sont situées au plus près de la marque (TC) sur deux côtés dans la direction de transport.

9. Procédé de fabrication d'un élément de type feuille pour un article absorbant selon la revendication 8, dans lequel
une taille (LTC) de la marque (TC) dans la direction de transport est inférieure à une distance (Dh) dans la direction de transport entre, parmi la pluralité de parties formant trou (h), la paire de parties formant trou (h) qui sont situées au plus près de la marque (TC) sur les deux côtés dans la direction de transport.

10. Procédé de fabrication d'un élément de type feuille pour un article absorbant selon l'une quelconque des revendications 1 à 9, dans lequel
les parties formant trou (h) sont des trous traversants qui passent au travers de la feuille composite dans la direction allant dans le sens de l'épaisseur.

11. Procédé de fabrication d'un élément de type feuille pour un article absorbant selon l'une quelconque des revendications 1 à 9, dans lequel
les parties formant trou (h) sont des trous à fond plat qui ont chacun une partie formant fond.

12. Dispositif de fabrication d'un élément de type feuille (30mf) pour un article absorbant (1) à partir d'une feuille composite,
l'élément de type feuille (30mf) ayant une région d'extensibilité élevée (AH) et une région de faible extensibilité (AL) côte à côte dans une direction prédéterminée,
la région de faible extensibilité (AL) ayant une extensibilité plus faible dans la direction prédéterminée par rapport à la région d'extensibilité élevée (AH),
une pluralité de parties formant trou (h) étant formées dans au moins la région d'extensibilité élevée (AH),
le dispositif comportant :
un dispositif de transport servant à transporter la feuille composite dans une direction de transport qui est la direction prédéterminée,
la feuille composite étant constituée par une première feuille de tissu non tissé (30mf1), une deuxième feuille de tissu non tissé (30mf2), et une pluralité d'éléments élastiques (35a),
la première feuille de tissu non tissé (30mf1) étant continue dans la direction prédéterminée,
la deuxième feuille de tissu non tissé (30mf2) étant continue dans la direction prédéterminée et étant agencée de manière à chevaucher la première feuille de tissu non tissé (30mf1) dans une direction allant dans le sens de l'épaisseur de la feuille composite,
les éléments élastiques de la pluralité d'éléments élastiques (35a) étant étirés dans la direction prédéterminée et s'étendant le long de la direction prédéterminée,
les éléments élastiques de la pluralité d'éléments élastiques (35a) étant insérés entre la première feuille de tissu non tissé (30mf1) et la deuxième feuille de tissu non tissé (30mf2) selon un agencement côte à côte dans une direction allant dans le sens travers et étant fixés dans au moins une région (AH1) correspondant à la région d'extensibilité élevée (AH),
la direction allant dans le sens travers étant une direction qui croise la direction prédéterminée ;
un dispositif de formation de parties formant trou (50) servant à former des parties formant trou (h) dans la feuille composite dans la région correspondant à la région d'extensibilité élevée (AH), et à mettre en prise la première feuille de tissu non tissé (30mf1) et la deuxième feuille de tissu non tissé (30mf2) l'une par rapport à l'autre dans au moins des parties formant bord (he) des parties formant trou (h),
les parties formant trou (h) s'étendant dans la direction allant dans le sens de l'épaisseur,
la formation des parties formant trou (h) étant effectuée en enfonçant un élément d'enfoncement (55p) dans la direction allant dans le sens de l'épaisseur au niveau de positions entre une paire d'éléments élastiques (35a) qui sont adjacents dans la direction allant dans le sens travers et qui sont dans au moins la région (AH1) correspondant à la région d'extensibilité élevée (AH) ; et
un dispositif de découpe d'éléments élastiques (80, 90) servant à découper la pluralité d'éléments élastiques (35a) dans une région (AL1) correspondant à la région de faible extensibilité (AL), pour former la région d'extensibilité élevée (AH) et la région de faible extensibilité (AL) côte à côte dans la direction de transport dans la feuille composite,
la découpe étant effectuée au niveau d'une position qui est en aval par rapport à une position de formation de parties formant trou (P51) dans la direction de transport,
les éléments élastiques (35a) étant empêchés d'être visibles au travers des parties formant trou (h), et
la position de formation de parties formant trou (P51) étant une position au niveau de laquelle la formation des parties formant trou (h) par le dispositif de formation de parties formant trou (50) est effectuée.
